# EUROPEAN PATENT APPLICATION

(11) **EP 3 425 586 A1**
(43) Date of publication of application: **09.01.2019**
(21) Application number: 17760316.4
(22) Date of filing: 02.03.2017
(51) Int. Cl.: G06Q 50/22, G06Q 50/10, G06Q 20/38, G06F 19/00, H04W 4/12, H04L 12/58

(54) **APPARATUS AND METHOD FOR ASSISTING IN MEDICAL CONSULTATION**

(30) Priority: 02.03.2016 KR 20160024970
(71) Applicant: Ikoob Co.,Ltd, Seoul 06591 (KR)
(72) Inventor: CHO, Jae Hyoung, Seoul 06715 (KR); JU, Ji Hyeon, Seoul 06601 (KR)
(74) Representative: Zardi, Marco
(86) International application number: PCT/KR2017/002264
(87) International publication number: WO 2017/150912

(57) **Abstract**

A medical consultation support method is disclosed. The method includes: enabling a consultant to gain access to a medical consultation support program; searching and selecting an image necessary to draft medical consultation content; displaying a drafting page of the medical consultation content loaded with the selected image; receiving input data of the consultant to be inserted in the drafting page of the medical consultation content; generating the medical consultation content through a combination of the received input data and the drafting page of the medical consultation content; and providing a consulter with the generated medical consultation content.

## Description

### [Technical Field]

The present disclosure relates to device and method of supporting a medical consultation. And more particularly, the present disclosure relates to device and method which are adapted to display a content associated with the medical consultation, generate a medical consultation content using an input of a consultant, and provide the generated medical consultation content to a consulter.

### [Background Art]

A medical field treats the life of a living organism (or living thing). As such, the medical field is an extremely specialized field. Therefore, the medical consultation has different aspects from general consultations.

The medical consultation is subject to restrictions and limitations due to particularity of a medical treatment. However, the medical consultation largely contributes to widening the width of mutual comprehension and eliminating (or lowering) a hospital doorsill for patients. Also, the medical consultation is being tried in a variety of types using information and communication technologies. Recently, information and communication appliances are generally being used. As such, medical institutions prepare consultation channels of various types and provide medical information through the prepared consultation channels. Moreover, the medical institutions open internet bulletin boards for the medical consultation and answer to questions of patients through the opened internet bulletin board. In other words, various remote medical consultations, such as medical consultations via telephone and a variety of internet media, are recently being tried by medical institutions.

Meanwhile, the publication of medical information and an easy access to the medical information using communication media are highlighted in an affirmative side capable of eliminating an information asymmetry problem but inundate wrong medical information. The inundation of the wrong medical information causes not only a direct meeting of a patient and a medical personal to be impeded but also possibility of causing an information distortion between both the patient and the medical personal to exist.

In general, the patient most prefers to have the medical consultation through a direct meeting with a doctor. However, the particularity of the medical field causes specialized medical information of the doctor not to be easily transferred to the patient.

Korean publication patent number 10-2011-0134666, as a prior art related to the remote medical consultation, discloses method, service server and system of providing a medical consultation service using a digital pen. The method disclosed in Korean publication patent number 10-2011-0134666 enables not only an applicant to apply the medical consultation to a remote answerer, such as a doctor, a specialist or others, using the digital pen but also the answerer to give an answer to the applicant about the applied medical consultation. However, Korean publication patent number 10-2011-0134666 realizes only mutual understanding between a doctor and a patient through a computer system but has a limitation without presenting a special technical model which raises the worth of opinion exchange in accordance with increased reliability of a recorded document about the mutual opinion exchange. Moreover, because only the remote medical consultation without the direct meeting between the doctor and the patient is performed, Korean publication patent number 10-2011-0134666 is difficult to largely deviate from existing manners of transferring the medical information through the media communication.

As another prior art related to the remote medical consultation, Korean Publication Patent No. 10-2013-0089911 proposing a marketing method using a hospital chart (or medical chart) is disclosed. The marketing method relates to only a technology being easy to manage the hospital chart in which a diagnosis of a doctor is completed, but does not introduce any technical model capable of increasing the comprehension of the patient for the medical consultation. Moreover, the hospital chart is one of documents being not provided to the patient. Due to this, the marketing method disclosed in Korean Publication Patent No. 10-2013-0089911 is limited to only increment of management efficiency of the hospital in applicable fields, cannot be applied to increment of medical consultation efficiency.

### [Prior Art Documents]

### [Patent Art Documents]

(Patent Document 1) Korean Publication Patent No. : 2011-0134666
(Patent Document 2) Korean Publication Patent No. : 2013-0089911

### [Disclosure]

### [Technical Problem]

Accordingly, embodiments of the present disclosure are directed to medical consultation support system and method that substantially obviate one or more of problems as described above.

The embodiments relate to provide medical consultation support system and method that are adapted enhance satisfaction for a medical consultation.

The embodiments relate to provide medical consultation support system and method that are adapted to promote the quality of medical consultation and understanding of a consulter for the medical consultation by sequentially provide a variety of audiovisual materials, which support (or assist) the medical consultation, in accordance with medical examination and treatment procedures at the medical consultation.

The embodiments relate to provide medical consultation support system and method that are adapted to generate a high quality of medical consultation content for a consulter by preparing a graphical user interface which inputs audiovisual materials and consultation substances (or contents) of a consultant in various modes.

The embodiments relate to provide medical consultation support system and method which are adapted to apply a generated medical consultation content to a consulter in on/off line modes so that not only the consulter checks one's own medical treatment substances (or contents) and shares the medical treatment substances with the other people but also a consultant is motivated to increase the quality of medical treatment.

The embodiments relate to provide medical consultation support system and method that are adapted to enable a consulter not only to receive a printed material including medical consultation substances (or contents), medicine instructions, and the proper use of an unfamiliar medicine injector but also to continuously remember one's own medical treatment substances (or contents).

The embodiments relate to provide medical consultation support system and method that can allow a consulter to have helps from intimate persons by sharing medical consultation substances (or contents) with the intimate person through a social network.

The embodiments relate to provide medical consultation support system and method that are adapted to ascertain at a time not only an expense being caused by the application of a medical treatment method, medicines and so on necessary for a medical treatment of diseases but also applicability of insurances therefor, and decide a reasonable application of the medical treatment method, the medicines and so on.

The embodiments relate to provide medical consultation support system and method that are adapted to perform a medical consultation using a variety of contents and to improve a circumstance of medical examination and treatment.

The embodiments relate to provide medical consultation support system and method that are adapted to actively guide pharmaceutical companies to create a variety of contents related to medicine development and pharmaceutical information.

The embodiments relate to provide medical consultation support system and method that are adapted to prevent an illegal and unreasonable application of insurance by enabling an insurance company to check consultation details between a consulter and a consultant, which corresponds to a medical specialist, and signatures of the consulter and the consultant.

### [Technical Solution]

A medical consultation support method according to an aspect of embodiments includes: enabling a consultant to gain access to a medical consultation support program; searching and selecting an image necessary to draft a medical consultation content; displaying a drafting page of the medical consultation content loaded with the selected image; receiving input data of the consultant to be inserted in the drafting page of the medical consultation content; generating the medical consultation content through a combination of the received input data and the drafting page of the medical consultation content; and providing a consulter with the generated medical consultation content.

Also, a medical consultation support terminal according to an aspect of embodiments includes: a touch screen configured to display graphic images and receive input data; a processor configured to execute a medical consultation support program; and a memory configured to store commands which will be executed by the processor. The processor controls: a consultant to gain access to the medical consultation support program; a graphical user interface searching and selecting an image necessary for a draft of a medical consultation content to be provided through the touch screen; a drafting page of the medical consultation content with a selected image to be displayed on the touch screen; the input data, which will be inserted in the drafting page of the medical consultation content, to be received from the consultant through the touch screen; the medical consultation content to be generated by combining the input data of the consultant and the drafting page of the medical consultation content; and the generated medical consultation content to be transferred to a consulter.

### [Effects of the Invention]

As described above, the medical consultation support method and terminal in accordance with embodiments of the present disclosure can promote satisfaction of a consulter, such as a patient, for a medical consultation with a consultant.

In detail, the medical consultation support method and terminal in accordance with embodiments of the present disclosure can promote quality of a medical consultation and understanding of a consulter for the medical consultation by sequentially provide a variety of audiovisual materials, which support (or assist) a medical consultation, in accordance with medical examination and treatment procedures at the medical consultation. In other words, the specialized knowledge and unfamiliar medical terms can be easily explained to patients. As such, the medical consultation support method and terminal can induce the patient to easily understand one's own disease and actively try to overcome one's own disease.

More specificationally, the medical consultation support method and terminal in accordance with embodiments of the present disclosure can generate a high-quality medical consultation content for a consulter by preparing a graphical user interface which inputs audiovisual materials and consultation substances (or contents) of a consultant in various modes.

The medical consultation support method and terminal in accordance with embodiments of the present disclosure can apply a generated medical consultation content to a consulter in on/off line modes so that not only the consulter checks one's own medical treatment substances (or contents) and shares the medical treatment substances (or contents) with the other people but also a consultant is motivated to increase the quality of the medical treatment. As such, a consulter can not only receive a printed material including medical consultation substances (or contents), medicine instructions, and the proper use of an unfamiliar medicine injector but also continuously remember one's own medical treatment substances (or contents) . Also, the consulter to have helps from intimate persons by sharing the medical consultation content with the intimate person through a social network. Moreover, the consulter can ascertain at a time not only an expense being caused by the use of a medical treatment method, medicines and so on necessary for a medical treatment of diseases but also applicability of insurances therefor, and decide a reasonable application of the medical treatment method, the medicines and so on.

Also, the medical consultation support method and terminal in accordance with embodiments of the present disclosure can enable a medical treatment method together with causable advance reactions thereby to be sufficiently transferred to consulters. As such, a medical service difficult for a patient to recover a prior state after one try can be more carefully selected.

In addition, the medical consultation support method and terminal in accordance with embodiments of the present disclosure can allow a consultant, such as a doctor, to explain specialized medical knowledge to a consulter using a variety of content. As such, a circumstance of medical examination and treatment can be improved and satisfaction of a patient can be increased. Therefore, it can be a great help for the consultant to attract patients. Also, necessary information, such as the specialized medical knowledge and so on, can be more easily and efficiently transferred to the patient.

Moreover, the medical consultation support method and terminal in accordance with embodiments of the present disclosure can enable a medical consultation to proceed in a direction helping the consulter with a reasonable selection without dazzling the consulter through the use of specialized terms, in consideration of a feature of medical service pertaining in a very specialized field and difficulties in full understanding of the consulter for medical information.

Further, the medical consultation support method and terminal in accordance with embodiments of the present disclosure can allow pharmaceutical companies to unaffectedly publicize information about directions and effects of their own medicines and so on through an opinion exchange between a consultant and a consulter without simply publicizing their medicines and medicine injection appliances. As such, the medical consultation support method and terminal can guide pharmaceutical companies to actively create a variety of content related to medicine development and pharmaceutical information. Therefore, benefits of the pharmaceutical companies can be increased and a medical consultation circumstance between the consultant and the consulter can be improved.

Furthermore, the medical consultation support method and terminal in accordance with embodiments of the present disclosure can previously prevent illegal and unreasonable insurance application and insurance benefit calculation by enabling an insurance company to check consultation details between a consulter and a consultant, which corresponds to a medical specialist, and signatures of the consulter and the consultant. Such medical consultation details can be used in development of insurance produce except that they are used as basic materials for the insurance application and the insurance benefit calculation. As such, the medical consultation support method and terminal can enable insurance policies and products, which promote public and private benefits of the pharmaceutical companies and improve a circumstance of the medical consultation between the consultant and the consulter, to be actively developed.

Finally, the medical consultation support method and terminal in accordance with embodiments of the present disclosure can induce an active conversation between the consultant and the consulter except that it is provided the medical information. In accordance therewith, it can be largely promoted understanding between both the consultant and the consulter.

### [Brief Description of the Drawings]

Figure 1 is a block diagram showing an internal configuration of a medical consultation support device in accordance with an embodiment of the present disclosure.
Figure 2 is a block diagram showing a system of performing a medical consultation support method in accordance with an embodiment of the present disclosure.
Figure 3 is a diagram showing a memory map of a member management database in accordance with an embodiment of the present disclosure.
Figure 4 is a diagram showing a memory map of an organ database in accordance with an embodiment of the present disclosure.
Figure 5 is a diagram showing a memory map of a medical prescription database in accordance with an embodiment of the present disclosure.
Figure 6 is a diagram showing a memory map of a signature information database in accordance with an embodiment of the present disclosure.
Figure 7 is a diagram showing a memory map of an insurance information database in accordance with an embodiment of the present disclosure.
Figure 8 is a block diagram showing a functional internal configuration of the terminal which is used for the performance of the medical consultation method in accordance with an embodiment of the present disclosure.
Figure 9 is a flow chart illustrating step by step a medical consultation support method according to an embodiment of the present disclosure.
Figure 10 is a photograph showing an example of a log-in page which is provided by a medical consultation program.
Figure 11 is a graphical diagram illustrating a function of automatically listing up information about consulters.

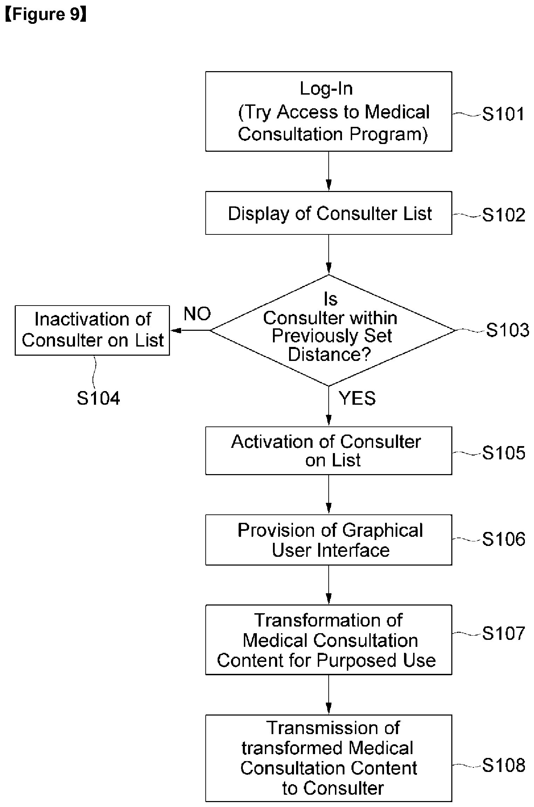

Figure 9 is a flow chart illustrating step by step a medical consultation support method according to an embodiment of the present disclosure.

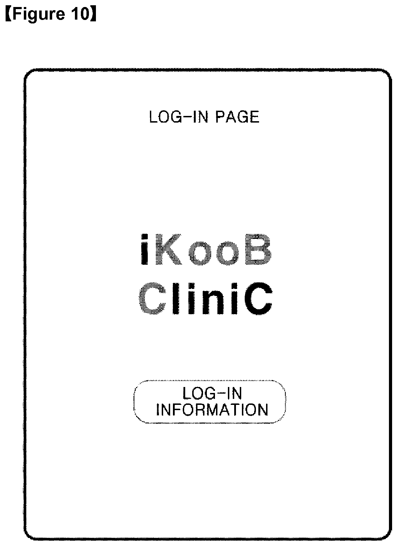

Figure 10 is a photograph showing an example of a log-in page which is provided by a medical consultation program.

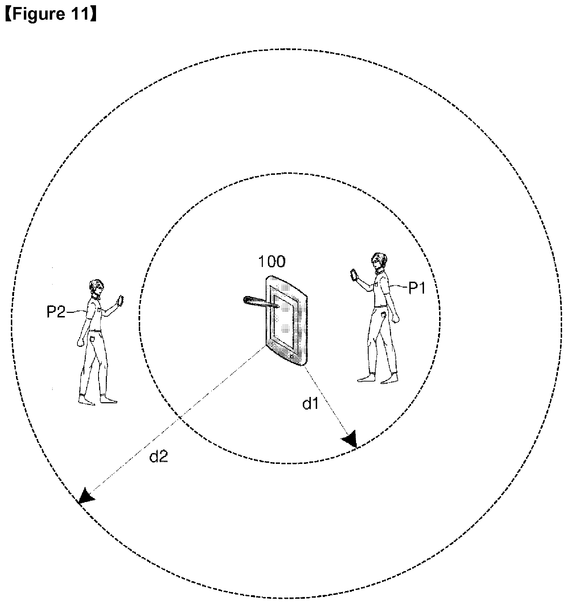

Figure 11 is a graphical diagram illustrating a function of automatically listing up information about consulters.

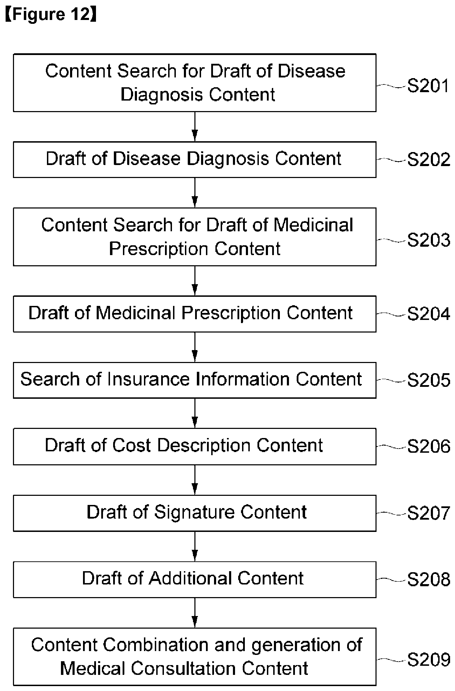

Figure 12 is a detailed flow chart illustrating a medical consultation content generation procedure in Figure 9.

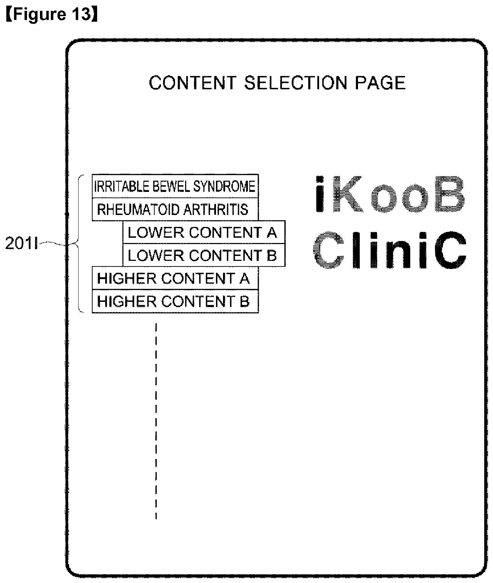

Figure 13 is a photograph showing an example of a content selection page which is necessary to draft a disease diagnosis content.

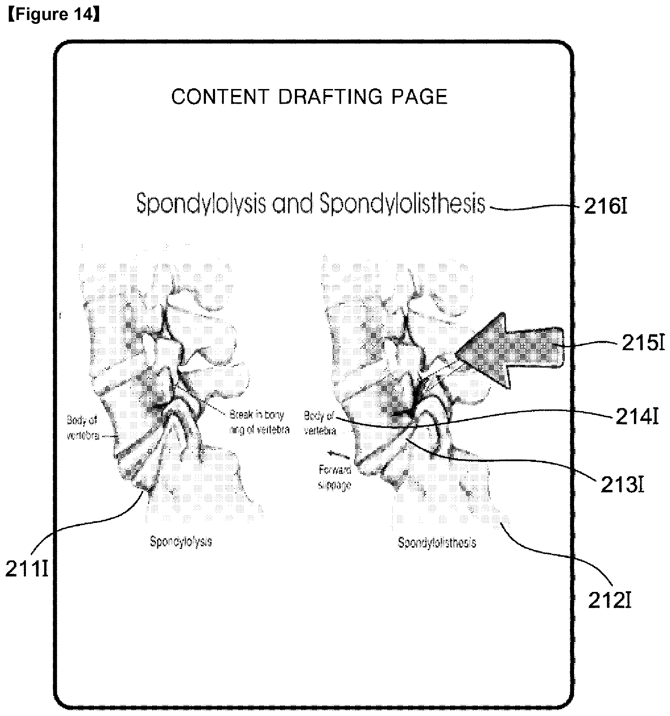

Figure 14 is a photograph showing an example of a drafting page of disease diagnosis content.

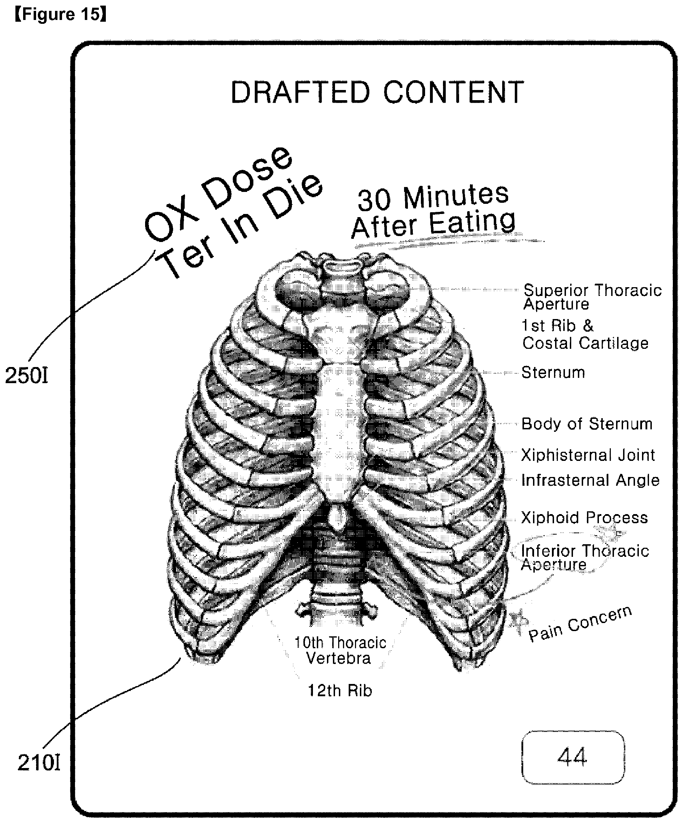

Figure 15 is a photograph showing an example of a drafted disease diagnosis content.

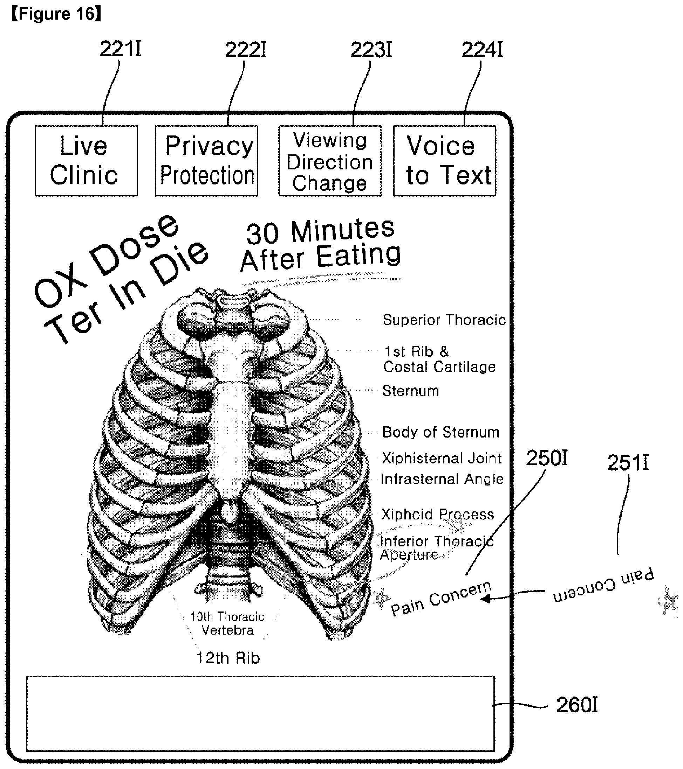

Figure 16 is a photograph showing another example of a drafting page of disease diagnosis content.

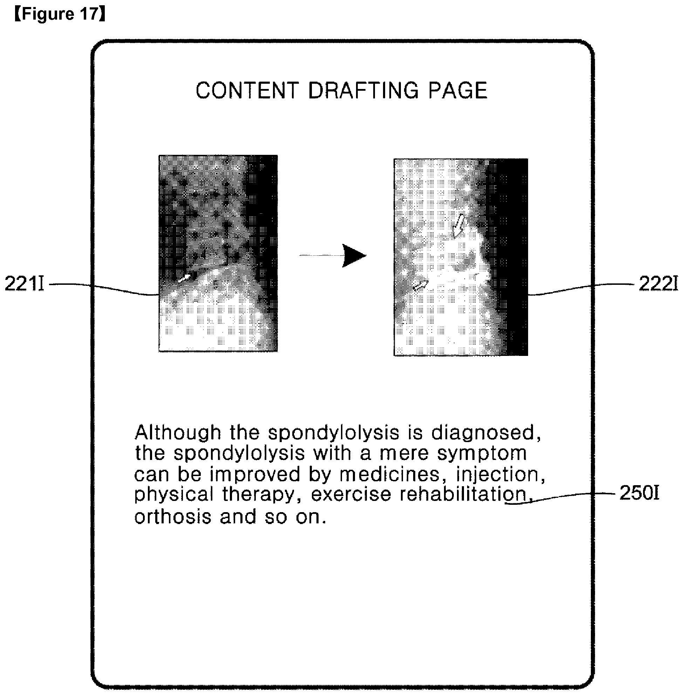

Figure 17 is a photograph showing an example of a drafting page of medical treatment method consultation content.

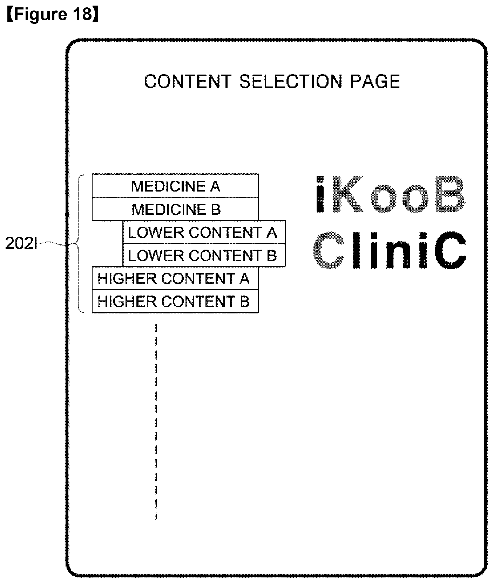

Figure 18 is a photograph showing an example of a content selection page necessary to draft medicine consultation content.

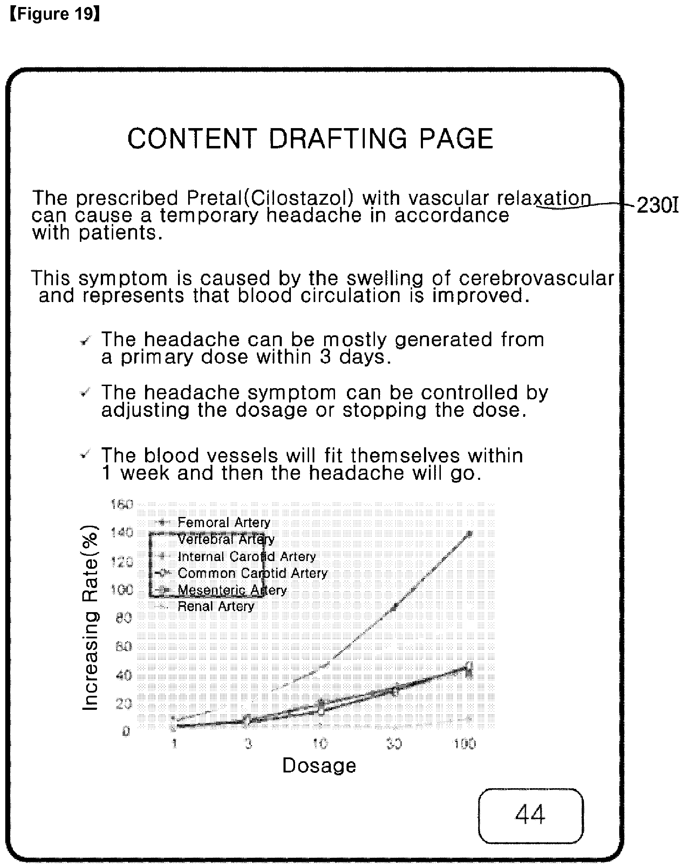

Figure 19 through 23 are photographs each showing examples of drafting pages of medicinal consultation content.

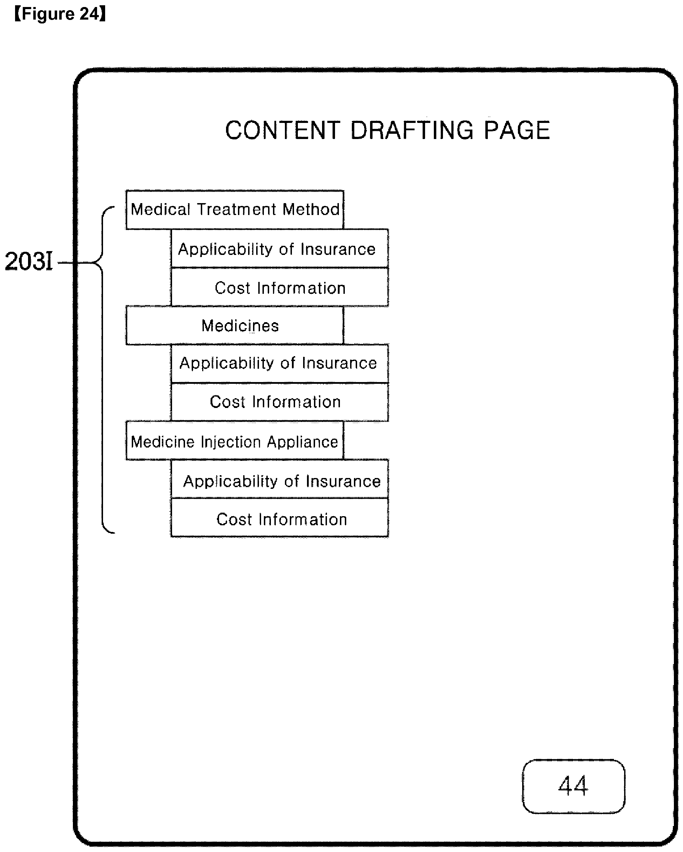

Figure 24 is a photograph showing an example of a drafting page of cost description content.

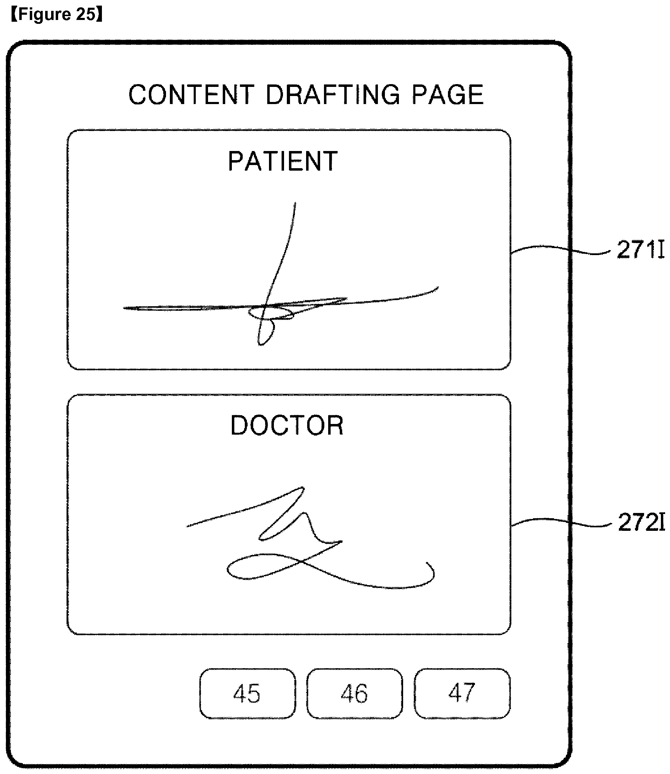

Figure 25 is a photograph showing an example of a drafting page of signature content which will be used for the verification of a medical consultation.

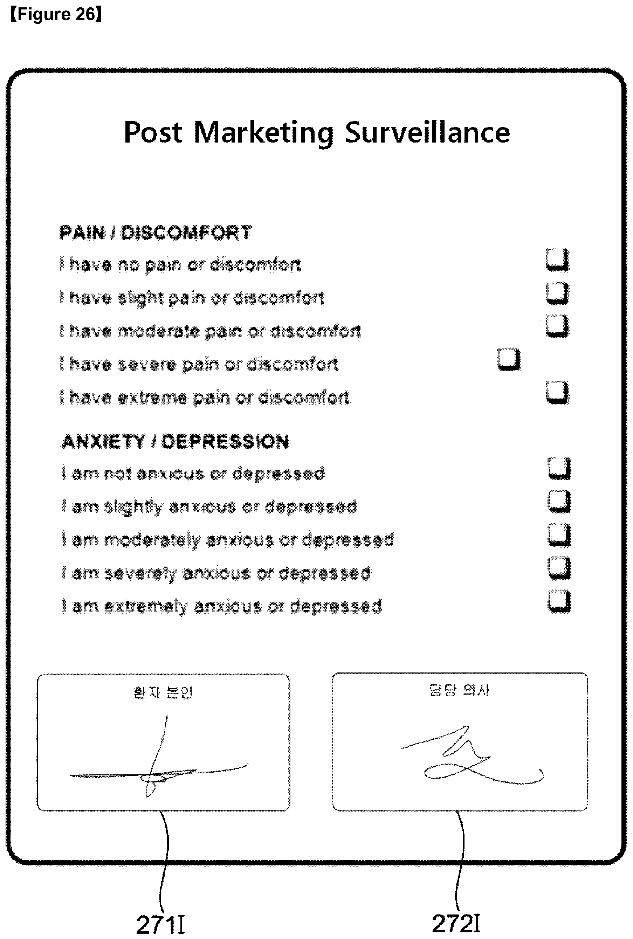

Figure 26 is a photograph showing an example of a drafting page of survey content for post marketing surveillance.

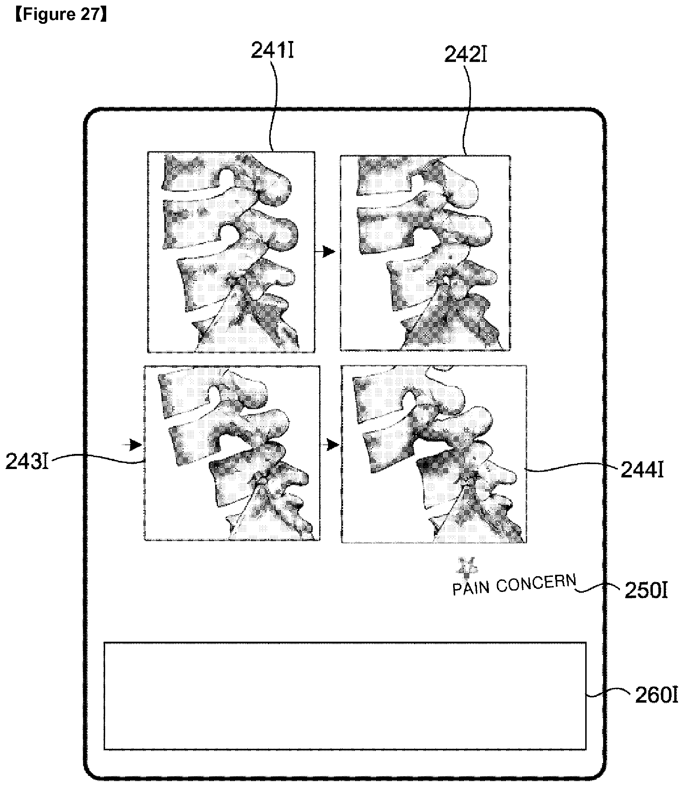

Figure 27 is a photograph showing an example of a printable medical consultation content derived from a medical consultation content.

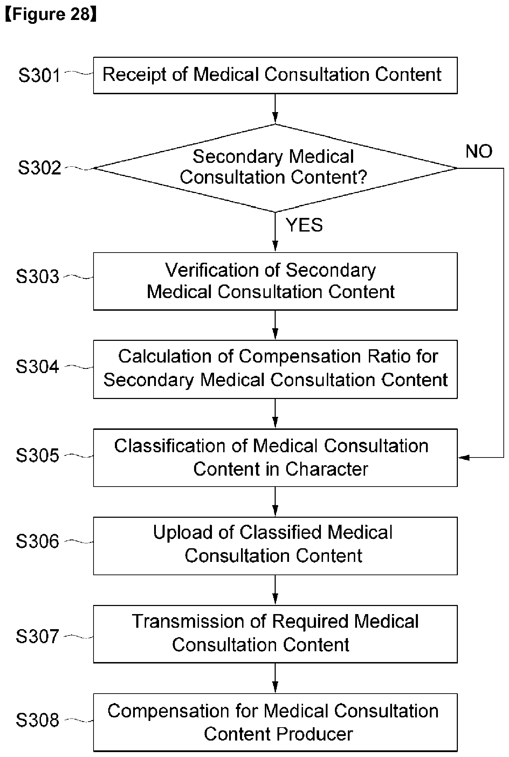

Figure 28 is a flow chart illustrating an operation procedure of an operating server which performs a medical consultation support method.

### [Detailed Description of the Embodiments]

Embodiments of the present disclosure will be shown in the drawings and described in detail through a detailed description. However, the present disclosure may be variously modified and embodied in a variety of different embodiments. Advantages and features of the present disclosure and implementation methods thereof will be clarified through the following embodiments described with reference to the accompanying drawings. Therefore, the present disclosure is not limited to these embodiments introduced hereinafter and might be embodied in a different shape from these embodiments.

The terms of first, second and so on in the present disclosure are used for distinguishing one component from the other components, but they do not specify limited meanings . Also, the singular forms used in the present disclosure are intended to include the plural forms, unless the context clearly indicates otherwise. Moreover, the terms "comprises" and/or "having" described in the present disclosure specify the presence of stated components and/or features, but do not preclude the presence or addition of one or more other components and/or features. Furthermore, the size or the thickness of each component in the drawings can be exaggerated or reduced for the definiteness of explanation. For example, the size and the thickness of each component in the drawings are arbitrarily represented for the convenience of explanation. In accordance therewith, the present disclosure is not limited to the matters shown in the drawings.

Reference will now be made in detail to the embodiments of the present disclosure with reference to the accompanying drawings. Wherever possible, the same reference numbers will be used throughout the disclosure including the drawings to refer to the same or like parts. As such, the repeatable description of the same or like parts will be omitted.

A terminal used as a device performing a medical consultation support method in accordance with an embodiment of the present disclosure can become one of a consol, a client computer, a server computer and a laptop computer modified therefrom. For example, at least one of a smart phone, a mobile phone, a laptop computer, a digital broadcasting terminal, a personal digital assistants (PDA), a portable multimedia player (PMP), a navigation device, a tablet personal computer, an ultra-book computer, a wearable device and a smart glass can be used as the terminal.

Such a terminal may be applied to all a terminal of a consultant, such as a doctor, a pharmacist, or a veterinarian, a terminal of a consulter, a terminal of an insurance manager and a terminal of a raw content provider. The terminal will now be described in detail.

### <Configuration of Terminal>

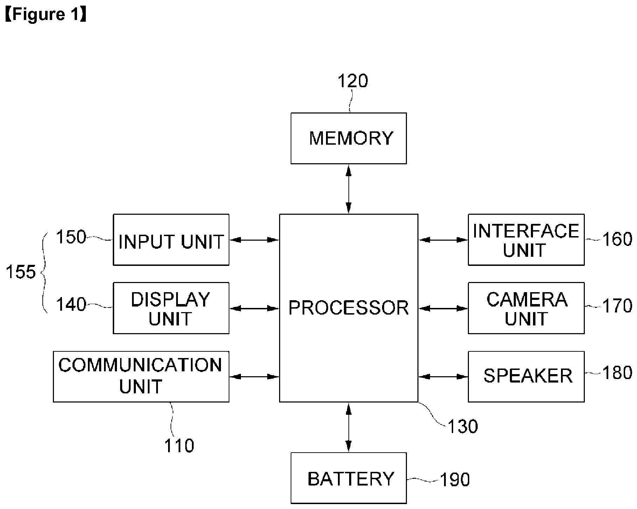

Figure 1 is a block diagram showing an internal configuration of a medical consultation support device in accordance with an embodiment of the present disclosure.

In detailed configuration, a terminal 100 in accordance with an embodiment of the present disclosure may include a communication unit 110, a memory 120, a processor 130, a display unit 140, an input unit 150, an interface unit 160, a camera unit 170, a speaker 180 and a battery 190 as shown in Figure 1. All the components shown in figure 1 may be not necessary to implement the terminal 100. In other words, the terminal 100 described in this description can include components more or less than the number of the above-mentioned components.

The terminal 100 may include the communication unit 110 configured to communicate with a communication system, such as a server, a different terminal 100 of another person or the others, in a wireless communication mode.

In the embodiment, the communication unit 110 may communicate with an operating server and transmit and receive various data which are necessary to perform the medical consultation support method. In detail, the communication unit 110 can receive a medical consultation program, which is necessary to perform the medical consultation support method, from the operating server. Also, the communication unit 110 can receive a variety of medical consultation-related content which are required in a procedure of performing the medical consultation support method, from the operating server.

The medical consultation-related content may include audiovisual material of assisting (or supporting) the medical consultation. For example, the medical consultation-related content can include a disease diagnosis content, a medical treatment method content, a medicinal consultation content, medical treatment cost content, an insurance information content, a signature content, the other types of content and a medical consultation content unifying them.

Also, the medical consultation-related content can include at least one of body organ images, disease information, medical treatment method images, medical treatment method information, medicine dosing methods, medicine information and insurance information. Moreover, the medical consultant-related content can include auditory information which is used to generate the medical consultation content. Actually, drafting pages of the medical consultation content including visual images may be provided (or displayed) and input data of a user can be add (or inserted) to the drafting page of the medical consultation content. In accordance therewith, the medical consultation content can be formed. The visual images can be regarded as raw content.

In addition, the communication unit 110 can receive registration-patient-related information, insurance-related information, post marketing surveillance (PMS) information, signature information and so on from the operating server, a hospital EMR (Electronic Medical Record) system and a home EHR (Electronic Health Record) system. The home EHR system can continuously measure the state of a patient at home and record the measured information.

Moreover, the communication unit 110 can transmit the medical consultation-related content, which is drafted (or created) by the user, to the operating server.

Such a communication unit 110 can transmit and receive wireless signals to and from at least one of a cell site (or a cell tower), an external terminal and the server through a mobile network. The mobile network can be constructed along a mobile communication technology standard or a mobile communication mode. For example, the mobile network can be constructed using one of GSM (Global System for Mobile communication), CDMA (Code Division Multi-Access), HSDPA (High Speed Downlink Packet Access), HSUPA (High Speed Uplink Packet Access), LTE (Long Term Evolution), LTE-A (Long Term Evolution-Advanced) and so on.

In another manner, the communication unit 110 can transmit and receive the wireless signals in a wireless communication mode, such as one of WLAN (Wireless Local Area Network), Wi-Fi (Wireless-Fidelity), Wi-Fi Direct, DLNA (Digital living Network Alliance), WiBro (Wireless Broadband), WiMAX (World Interoperability for Microwave Access) and so on.

Furthermore, the communication unit 110 can obtain the position (or present position) information of the terminal 100. To this end, the communication unit 110 can include one of a GPS (Global Positioning System) module and a WiFi (Wireless Fidelity) module.

The terminal 100 may further include: the memory 120 configured to store a medical consultation program, the medical consultation-related content, protocols and so on; and the processor 130 configured to execute various programs stored in the memory 120 and perform arithmetic and control operations.

In detail, the memory 120 can store a variety of application programs or application software being driven in the terminal 100, and data and commands related to the operations of the terminal 100.

In the embodiment, the memory 120 can also store the medical consultation program, previously used medical consultation-related content, patient information, a plurality of medical consultation content of existing patients and so on.

The application program may be installed in the terminal 10 in such a manner as to be stored in the memory 120. The application program may be driven (or executed) by the processor 130. In accordance therewith, the terminal 100 can perform given (or primary) operations (or functions).

Such a memory 120 can be configured in a hardware mode. In this case, the memory 120 can include at least one of several storage devices such as ROM (Read Only Memory), RAM (Random Access Memory), EPROM (Erasable Programmable Read Only Memory), flash drive, hard drive and so on. In another manner, the memory 120 can become a web storage having a storage function.

The processor 130 may generally control operations of the components within the terminal 100 and provide a medical consultation support function.

In the embodiment, the processor 130 performs various medical consultation support functions through execution of the medical consultation program stored in the memory 120.

In detail, the processor 130 performing the medical consultation support functions may control a variety of audiovisual support material supporting (or assisting) the medical consultation to be displayed. In this case, the processor 130 may provide a user interface which is necessary for a consultant to search and select the audiovisual support materials.

The processor 130 may also supply the consultant with a graphical user interface which is used to generate the medical consultation content. Moreover, the processor 130 may control a generated medical consultation content to be edited and/or output through at least one of online and offline.

Such a processor 130 may be implemented using at least one of ASICs (Application Specific Integrated Circuits), DSPs (Digital Signal Processors), DSPDs (Digital Signal Processing Devices), PLDs (Programmable Logic Devices), FPGAs (Field Programmable Gate Arrays), processor units, controllers, micro-controllers, micro-processors and electrical units performing the other functions.

The processor 130 may ordinarily control overall operation of the terminal 100 in the exception of the operations related to the application programs. Also, the processor 130 may process and/or apply information and/or functions, which are suitable for the user. To this end, the processor 130 may process signals, data, information and so on, which are received through different components within the terminal 100, and execute the application programs stored in the memory 120.

Also, the terminal 100 may include the display unit 140 configured to display graphic images.

The display unit 140 in accordance with the embodiment may display the medical consultation content and/or the drafting page thereof. Also, the display unit 140 may display the graphical user interface, which is necessary to make out the medical consultation, on its screen.

Such a display unit 140 may include at least one of a liquid crystal display (LCD) device, a thin film transistor - liquid crystal display (TFT-LCD) device, an organic light emitting diode (OLED) device, a flexible display device, a three-dimensional display device, an electronic ink display device (or an electrophoretic display device).

The terminal 100 may further include the input unit 150 configured to sense an input of the user.

The input unit 150 in accordance with the embodiment may receive input data of the user which will be used to generate the medical consultation content. For example, the input unit 150 may receive a content search/selection instruction, written data of the user, consultation voices of the user and so on, and transfer the received input data to the processor 130.

Also, the input unit 150 may receive an execution command for powering on/off the terminal 100 as well establishment and execution commands related to various functions of the terminal 100, from the user.

In order to receive the above-mentioned inputs from the user, the input unit 150 may include at least one of a gesture input portion, a touch input portion and a micro-phone. The gesture input portion may include an optical sensor or other and sense a gesture of the user. The touch input portion may sense a touch of the user. To this end, the touch input portion may include a touch sensor, a touch key, a mechanical key and so on. The microphone may sense a voice of the user.

In another manner, the display unit 140 and a touch sensor of the input unit 150 may be stacked in a multi-layer stacked structure and united with each other in a single body. In accordance therewith, the display unit 140 and the input unit 150 may implement a touch screen. The touch screen may provide a graphical user interface between the terminal 100 and the user and perform a function of an input/output module for the user.

The input/output module can become a term unifiedly calling the input unit 140 and the output portion. The input unit 140 can include a touch screen, input buttons, the camera unit 170 and a microphone. The output portion can include the touch screen, the speaker 180 and a vibrator (not shown) configured to vibrate the terminal 100.

In addition, the terminal 100 may include the interface unit 160 configured to receive external data and externally transfer signals which are processed or generated by the processor 130.

The interface unit 160 in accordance with the embodiment may transfer the medical consultation content to an external printer in order to print the medical consultation content as off-line output material.

The interface unit 160 of the terminal 100 of a consultant side may be connected to the terminal 100 of the consulter in one of a wire mode and a wireless mode and transmit the medical consultation content to the terminal 100 of the consulter. Also, the interface unit 160 may receive images, which photograph a medical treatment procedure, from an externally disposed camera (not shown).

In other words, the interface unit 160 can provide a communication path (or channel) with external appliances which are connected to the terminal 100. Such an interface unit 160 may include at least one of a wire/wireless headset port, an external charger port, a wire/wireless data port, a memory card port, a device port for an external device with an identification module, an audio input/output port, a video input/output port and an earphone port. If an external appliance is connected to the interface unit 160, the terminal 199 can perform proper control operations related to the connected external appliance.

Moreover, the terminal 100 may include the camera unit 170 configured to photograph at least one of medical treatment and consultation procedures.

In detail, the camera unit 170 can include a front camera and a rear camera. The front camera can be disposed on a front surface of the terminal 100 and photograph a forward subject. The rear camera can be disposed on a rear surface of the terminal 100 and photograph a backward subject.

In another manner, the camera unit 170 can be disposed at an external position which is separate from the terminal 100, the consultant and/or the consulter and easy to photograph the medical treatment and consultation procedures. In this case, the camera unit 170 can transfer the photographed images for the medical treatment and consultation procedures to the processor 130 through one of the communication unit 110 and the interface unit 160.

Such a camera unit 170 may include an image sensor, such as a CCD (Charge Coupled Device) sensor or a CMOS (Complementary Metal Oxide Semiconductor) sensor, and an image process module. The camera unit 170 can process still images and motion images which are obtained through the image sensor. In detail, the image process module can process the still images and/or the motion images which are obtained through the image sensor. Also, the image process module can extract necessary information from the processed images and transfer the extracted information to the processor 130.

Furthermore, the terminal 100 may include the speak 180 and the battery 190. The speaker 180 may convert the auditory content within the medical consultation content into audio and output the converted audio. The battery 190 may supply electric power to all the components within the terminal 100.

### < System of Performing Medical Consultation Support Method>

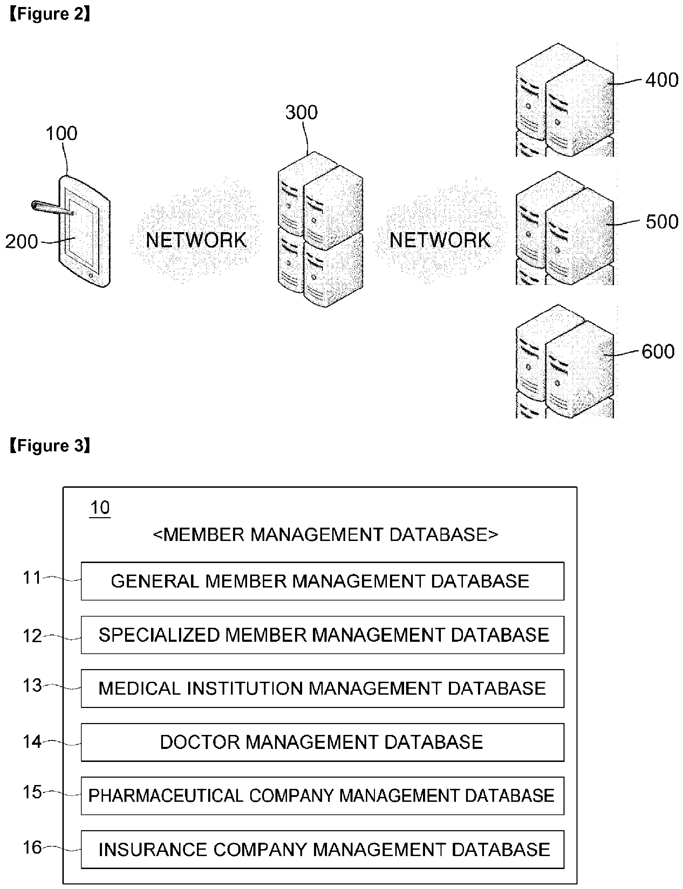

Figure 2 is a block diagram showing a system of performing a medical consultation support method in accordance with an embodiment of the present disclosure.

The system of performing the medical consultation support method may include: a terminal 100 configured to directly executing a medical consultation support function; an operating server 300 configured to provide the variety of medical consultation-related content necessary for a performance of the medical consultation support function; and external servers 400, 500 and 600 each configured to provide the variety of medical consultation-related content and the other information to the operating server. The external servers 400, 500 and 600 may include at least one of a medical information presentation server 400, a medicinal information presentation server 500 and an insurance information presentation server 600. The medical information presentation server 400 may provide the plurality of medical consultation content, disease consultation information and so on. The medicinal information presentation server 500 may provide a plurality of medicinal consultation content. For example, a pharmaceutical company server may be used as the medicinal information presentation server 500. The insurance information presentation server 600 may provide insurance-related information.

The terminal 100 may download and install a medical consultation program from the operating server 300 and execute the installed medical consultation program. In accordance therewith, the medical consultation support function can be performed or provided by the terminal 100.

In detail, the medical consultation program may be a medical consultation-purposed application program of inquiring the medical consultation-related content, which is necessary for the consultation and promotes understanding of a patient at a medical treatment of the patient, and providing a graphical user interface which receives the input data of the user by enabling the user to handwrite data on an inquired content. The medical consultation program may accurately transfer medical information which is necessary for the patient to manage one's own disease oneself and includes medical information of a preventive medicine purpose, information about patient's disease and so on, surgical treatment methods, medicine dosing methods, harmful effects of medicines and so. The written data may be inserted in the drafting pages of a note shape, which include a variety of content and is provided by the medical consultation program, using a touch function which is provided by the terminal 100.

Such a medical consultation program may be executed in the terminal 100 in response to a specified execution command by being received (or downloaded) from the external server to the terminal 100 through a network and installed in the terminal 100. The external server may become the operating server 300. In another manner, the medical consultation program stored in the operating server 300 can be uploaded in a different external server which manages downloads of application programs and so on. In this case, a plurality of users using the above-mentioned terminal 100 can download the medical consultation program from the different external server to their own terminals 100.

If the user is a consultant capable of providing medically relevant information, the consultant can download the medical consultant program and install the downloaded program in one' s own terminal 100 in order to provide medical information to the consulter. When the user is a consulter, the consulter can download the medical consultant program and install the downloaded program in one's own terminal 100 in order to read a medical consultation record. A pharmaceutical company providing medicine information and/or an insurance company can upload content using the medical consultation program as needed. To this end, the pharmaceutical company and/or the insurance company can download the medical consultation program and install the downloaded program in their own terminals 100.

The medical consultation program or the operating server 300 can distinguish whether an accessor is someone of a consultant, a consulter, a pharmaceutical company and an insurance company, on the basis of information of the accessor accessed through a 'log-in' operation. Also, the medical consultation program or the operating server 300 can selectively provide plural menus different from one another in accordance with a character of the accessor. The plural menus can be previously determined on the basis of information publication ranges which are differently set in accordance with the character of the accessor.

The operating server 300 may store a plurality of medical consultation programs distinguished along with the character of the accessor. The plurality of medical consultation programs maybe suitably programmed for each of the consultant, consulter, pharmaceutical company and insurance company and provide the variety of content distinguished from one another. In order to prevent an unauthorized outflow of various inherent information and content which are provided by the medical consultation program, the operating server 300 may disallow an access of the consulter or intercept a download of the consulter.

Also, the medical consultation program may be connected with an intranet within a hospital to which the consultant belongs and display a previously set menu shape suitable for a system of the respective hospital.

The specified execution command may become a program execution command of the user being input through the input/output module of the terminal 100. In order to input the program execution command to the terminal 100, the user may manipulate a power button of the input/output module of the terminal 100, press one of various buttons of the input/output module within the terminal 100, or touch the touch screen of the input/output module of the terminal 100.

Also, the special execution command may be generated when the terminal 100 is connected to previously set anyone among various kinds of networks through one's own communication unit 110. In a different manner, the special execution command may be generated when the user accesses to a previously set website through the terminal 100. In another different manner, if the terminal 100 includes a function of a global positioning system (GPS) capable of receiving position information, the special execution command may be generated when the terminal 100 is positioned at a previously set position.

In other words, the terminal 100 with the installed medical consultation program can perform a medical consultation content displaying function, a record receiving function, a verification function and an information transmission function. The operating server 300 in accordance with an embodiment of the present disclosure may be one of readable media such as a computer storing the medical consultation program.

Such an operating server 300 may include at least one database storing a variety of information, in order to provide the medical consultation support function. Principal information stored in the database may become the variety of medical consultation-related content. As such, the operating server 300 may store classifiedly a variety of audiovisual materials necessary to draft the medical consultant content, the plurality of drafted medical consultation content and so on in the database. Also, the operating server 300 may apply classification lists to the terminal 100 and transmit a single content which is selected by a search of the terminal 100 (or a search of the user).

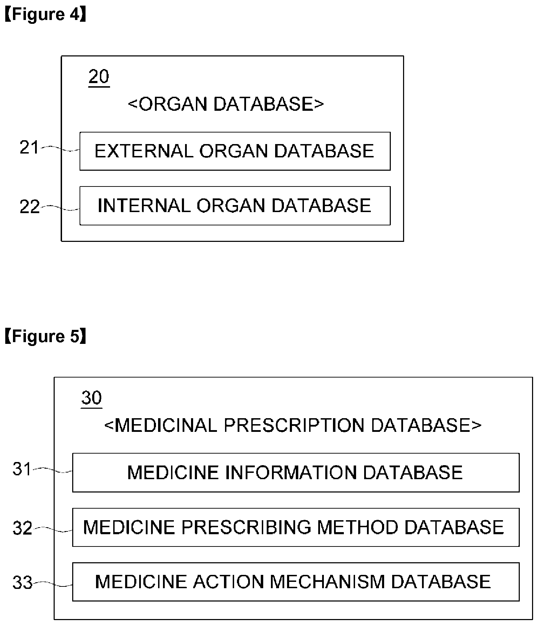

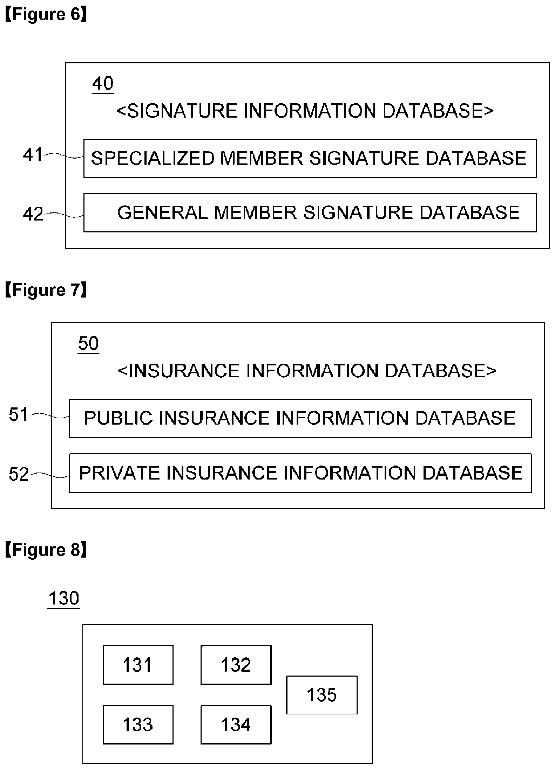

Figure 3 is a diagram showing a memory map of a member management database in accordance with an embodiment of the present disclosure. Figure 4 is a diagram showing a memory map of an organ database in accordance with an embodiment of the present disclosure. Figure 5 is a diagram showing a memory map of a medicinal prescription database in accordance with an embodiment of the present disclosure. Figure 6 is a diagram showing a memory map of a signature information database in accordance with an embodiment of the present disclosure. Figure 7 is a diagram showing a memory map of an insurance information database in accordance with an embodiment of the present disclosure.

### < Databases>

The operating server 300 may include a member management database 10, an organ database 20, a medical prescription database 30, a signature information database 40 and an insurance information database 50 which are configured to store a variety of information, in order to provide the medical consultation support function.

On the other hand, the memory 120 of the terminal 100 can receive at least a part of information from the databases of the operating server 300 and implement an individual database.

### - Member Management Database

The member management database 10 may store information about the users which use the medical consultation program. The user may become the member by registering oneself using the medical consultation program. The members registered in the member management database 10 may be classified into specialized members and general members. The specialized members may include doctors, pharmacists, oriental doctors, veterinarians and so on which have medical relevant qualifications and manage patients. Also, the specialized members may include professors and researchers of medical field capable of providing a variety of medical relevant information. The general members may include patients receiving a medicinal knowledge and a medical prescription from the specialized member. If an object of the medical consultation is an animal not human, the general members may include an authorized human for the management of the animal.

Also, the users may include medical institutions capable of performing medical treatment, prescription and consultation, pharmaceutical companies capable of providing a variety of medicines along with the medical prescription, and insurance companies being operated by national institutions and/or people.

The member management database 10 may classify the members based on the characters of the users and independently manage the classified members. To this end, the member management database 10 may include a general member management database 11 and a specialized member management database 12.

The general member management database 11 may include medical information of the patients. For example, the general member management database 11 may include information about age, sex, race, blood type, past disease history and past medical treatment history and so on of the patient.

Also, the general member management database 11 may receive and store the electronic health record (EHR) with weight, blood sugar level, blood pressure and so on of a patient which are measured at home of the patient.

The member management database 10 may further include at least one of a medical institution management database 13, a doctor management database 14, a pharmaceutical company management database 15 and an insurance company management database 16.

### - Organ Database

The organ database 20 may store information about a human body. In another manner, the organ database 20 may store information about the bodies of animals not the human body.

In detail, the organ database 20 may store information which includes entire and/or partial images for external organs of the human body and entire and/or partial images for internal organs of the human body. For example, the image information about the external organs of the human body may include entire images for an entire configuration of the external organs, such as a head, arms, legs, a trunk and so on. The image information about the external organs of the human body may further include entire and partial images for each of the external organs. Similarly, the image information about the internal organs of the human body may include entire images for an entire configuration of the internal organs, such as a skeletal system, muscles, blood vessels, viscera and so on. Also, the image information about the internal organs of the human body may further include entire and partial image for each of the internal organs and detailed components thereof.

Such an organ database 20 may include an external organ database 21 and an internal organ database 22, in order to distinguish a surgical treatment and an internal treatment.

The information stored in the organ database 20 may include a variety of organ content. The organ content may include an organ image and names (or titles) and/or descriptions for the detailed components forming the organ.

Also, an entire body and parts thereof, such as a face, a trunk, a leg, an arm and so on may have a variety of shapes in accordance with persons. For example, images of a body organ suitable for a plastic surgery consultation may be in need of faces of various shapes and/or detailed organs which have various shapes and form the face. As such, the organ database 20 may store a variety of organ content including body organ images of various shapes, which are classified on the basis of sex (men or women), age (infant, youngster, adult or oldster), sasang (or four typological) constitution (soeum, soyang, taeeum or taeyang topology), features of persons of each nation (oriental or occidental race) and feature of persons of each region, as well as body organ images of a normalized (or standardized) shape. The variety of organ content may further include image descriptions related to the respective body organ images. Therefore, proper body organ image matching physical features of the consulter can be selected and used at a consultation.

On the other hand, the consultant can generate shareable content, which is shared with the other persons through the organ database 20, or exclusive (or private) content only for one's own use. The generated content may be transferred to the operating server 300 and authenticated by the operating server 300. Such authenticated content can be used as regular content necessary for the consultation. The content authenticated as the regular content may correspond to an open source and be used by different consultants who meet requirements of the producer (or consultant). In another manner, the generated content can be provided as charge content. In this case, the operating server 300 may pay a writing point to the content producer (or consultant) at least one of when the authentication is completed and when the content is downloaded by a different consultant. Therefore, the content producer can be motivated to create high quality content suitable for the consultation.

### - Medicinal Prescription Database

The medicinal prescription database 30 may store information about a variety of medicines which are prescribed to the general member by the specialized member.

The medicinal prescription database 30 may include a medicine information database 31, a medicine prescribing method database 32 and a medicine action mechanism database 33. The medicine information database 31 may store information about names (or titles) and brief descriptions for a variety of medicines. The medicine prescribing method database 32 may store information about methods of prescribing a variety of medicines. The medicine action mechanism database 33 may store information about an action time and an adverse reaction of each medicine and attention matters at a medicine injection.

The information stored in the medicinal prescription database 30 may include a variety of content prepared by pharmaceutical companies which provide medicines and appliances necessary for a medicine injection, monitoring of the medicine injection and so on. Also, the information stored in the medicinal prescription database 30 may include a variety of content created by the consultant such as a medical specialist. The content may include an image and a description which are created by the medical specialist. In another manner, the content may include a text, a motion image, voices and so on which are created on the basis of knowhow of the medical specialist which is accumulated through medical treatment and consultation experiences. The text, motion image, voices and so on included in the content can be used throughout the medical consultation including explanations of effective medicine dosing method, medicine injection method, medicinal appliance handling method and so on. The content generated by the consultant can be in need of an authentication of the operating server 300. Also, the content generated by the consultant can be used as an open source, or receive a writing point.

### - Signature Information Database

The signature information database 40 may include a specialized member signature database 41 and a general member signature database 42. The specialized member signature database 41 may store signature information of the specialized members. The general member signature database 42 may store signature information of the general members.

### - Insurance Information Database

The insurance information database 50 may store insurance information related to whether or not a medical treatment, a surgery (or an operation), a medicine, a medicine injector and so on are applied to an insurance. Such an insurance information database 50 may include a public insurance information database 51 and a private insurance information database 52. The public insurance information database 51 may store the insurance information related to whether or not an application of a public insurance which is operated by a national (or public) institution. The private insurance information database 52 may store the insurance information related to whether or not an application of a private insurance which is operated by a private enterprise.

Also, the insurance information database 50 may be linked with the member management database 10 and store information about public and private insurances which are insured with each of the members.

### - Additional Database

The operating server 300 may further include an additional database with the exception of the above-mentioned databases. The additional database may store guide and disease content, surgical method content, adverse reaction content and medical treatment method content. The guide and disease content may include guide data for introducing disease-related knowledge, diet therapies and therapeutic exercises to the patients as well disease information about a variety of diseases. The surgical method content may include a variety of surgical methods related to diagnostic surgeries, eradicative surgeries, preventive surgeries and so on. The adverse reaction content may include information about adverse reactions due to surgeries. The medical treatment method content may include information about a variety of medical treatment methods.

The member management database 10 may store a medical consultation content related to a specific disease. The medical consultation content may include a disease diagnosis content, a medical treatment method content and a medicine consultation content which are unifiedly combined with one another in a previously set sequence.

The organ database 20 may store various content drafted (or created) by a principal agent (or an operating subject) of the operating server 300 and the consultants. Also, the organ database 20 may store a variety of additional content which are inserted into the existing content and displayed together with the existing content. The additional content may include images, such as a motion picture, an explanation material, an animation and so on introducing directions of a syringe. As such, the consultant can use the additional content for the medical consultation by inserting a necessary additional content into the displayed content. The content drafted (or created) by the consultant can need to receive an admission of the operating server 300. Also, the content drafted (or created) by the consultant may be in an open source shape or become content assigned with a writing point.

The member management database 10, the organ database 20, the medicine prescription database 30 and the signature information database 40 may be included in the operating server 300. As such, the terminal 100 may frequently download necessary data from the operating server 300 as needed. At least a part of data altered or modified by the executed medical consultation program may be transferred from the terminal 100 to the operating server 300. Also, as needed, the data transferred to the operating server 300 may be re-transferred from the operating server 300 to the terminal 100.

The medical consultation program may be used by a plurality of users. In this case, a plurality of terminals 100 may be prepared for the plurality of users, and so unique information about the respective users may be accumulated in the operating server 300. As such, only data permitted to the user of the terminal 100 connected to the operating server 300 may be transferred from the operating server 300 to the respective terminal 100.

The data stored in the organ database 20 may include data prepared in the operating server 300 and different data transferred from the medical information presentation server 400. The data transferred from the medical information presentation server 400 to the operating server 300 may be filtered by the operating server 300. The filtered data may be transmitted to the terminal 100 and stored in the organ database 20.

The medical information presentation server 400 may include a plurality of medical information presentation sub-servers which are operated by principal agents (or operations subjects) different from one another. The data about the organ information stored in each of the medical information presentation sub-servers may be choicely selected by the users . The principal agents (or operating subjects) may include public and private agencies which create images and descriptions therefor related to various organs of the human body (or each living body).

The medical information presentation server 400 applying the data about the medical information can be operated by the principal agents capable of providing specialized knowledge relevant to the medical field in an image shape. As such, the principal agents can include medical specialists able to become the consultant, pharmaceutical companies providing pharmaceutical information, and public and/or private organizations such as a variety of research parties, universities and so on. As such, the data about the medical information can be created (made) by a variety of principal agents. Therefore, it can be created or made a variety of the medical information images.

Meanwhile, the consultant can choicely select the medical data, which includes content and so on easy for the consulter to understand and suitable for the consultation and secures the accuracy of determination based on the specialized knowledge of the consultant, among the variety of medical data. In this case, the medical consultation program may enable information about the selected content to be transferred to the operating server 300. As such, the operating server 300 can classify the various selected content in manufacturing agents, count the selection quantity (or number) of each content, and calculate the writing point assigned to each of the medical information presentation sub-servers. Also, the operating server 300 can give (or pay) the respective medical information presentation sub-server the calculated writing point. Therefore, the medical consultation support system and method in accordance with an embodiment of the present disclosure can induce the principal agents of the medical information presentation sub-servers to quantitatively and qualitatively enhance their own content.

The data stored in the medicine prescription database 30 may include data created (or made) in the operating server 300 and different data provided from the medicinal information presentation server 500.

The data about the medicinal information may be transferred from the medicinal information presentation server 500 to the operating server 300. As such, the operating server 300 may filter the received data. The filtered data may be transferred from the operating server 300 to the terminal 100. Also, the filtered data may be stored in the medicine prescription database 30.

The medicinal information presentation server 500 may include a plurality of medicinal information presentation sub-servers which are operated by principal agents (or operations subjects) different from one another. The data about the medicinal information stored in each of the medicinal information presentation sub-servers may be choicely selected by the users. The principal agent (or operating subject) may become a pharmaceutical company producing and sells the medicines, as an example.

The medicinal information presentation server 500 applying the data about the medicinal information can be operated by the principal agents capable of providing specialized knowledge relevant to the medicines in an image shape. As such, the principal agents of the medicinal information presentation sub-servers can include a variety of pharmaceutical companies and public and/or private organizations such as research parties, universities and so on. As such, the data about the medicinal information can be created (made) by a variety of principal agents. Therefore, it can be created or made a variety of images about the medicinal information.

The medical specialist becoming the consultant may transmit content with medicinal information, which is created (or made) based on one's own knowhow, to the operating server 300 using the medical consultation program or one's own individual server. In this case, the individual server of the medical specialist may be used as a single kind of a medicinal information presentation sub-server in an expansive conception.

Meanwhile, the consultant can choicely select the medicinal data, which includes content and so on easy for the consulter to understand and suitable for the consultation and secures the accuracy of determination based on the specialized knowledge of the consultant, among the variety of data about the medicinal information. In this case, the medical consultation program may enable information about the selected content to be transferred to the operating server 300. As such, the operating server 300 can classify the selected content in content manufacturing agents, count the selection (or hit) number of each content, and calculate a hit ratio of each content and a selection quantity of respective medicine. The hit ratio of each content and the selection quantity of each medicine calculated in the operating server 300 may be transferred to the respective medicinal information presentation sub-server. The principal agent of the medicinal information presentation sub-server may also check (or confirm) the hit ratio of one' own content and the selection quantity of one's own medicine. In accordance therewith, the principal agents of the medicinal information presentation sub-servers may make an effort to promote the selection quantity (rank) of the consultant and consulter for their own produced medicine. Therefore, the medical consultation support system and method in accordance with an embodiment of the present disclosure can induce the principal agents of the medicinal information presentation sub-servers to quantitatively and qualitatively enhance their own content and medicines.

In other words, the medical consultation support system and method can motivate the principal agent of the medicine information presentation server 500 to enhance one's own company image and product brand image. Also, the medical consultation support system and method can enable the principal agent of the medicine information presentation server 500 to recognize that the principal agent of the medicine information presentation server 500 is being greatly contributed to the whole medical treatment circumstance of the consulter. Therefore, the medical consultation support system and method can actively induce the principal agent of the medicinal information presentation server 500 to qualitatively enhance one's own content and medicines and increase reliability in education, diagnosis and/or medical treatment related to the disease of the consulter.

Also, the medical consultation support system and method in accordance with an embodiment of the present disclosure can induce the principal agents of the medicinal information presentation server 500 to provide medicinal support functions necessary for a medical examination and treatment circumstance and a cost reduction benefit at a purchase of medicines, a medicine injection appliance or others. In accordance therewith, direct and indirect benefits can be applied to the consulter.

Figure 8 is a block diagram showing a functional internal configuration of the terminal 100 which is used for the performance of the medical consultation method in accordance with an embodiment of the present disclosure.

The terminal 100 executing the medical consultation program may be configured with the following functional components.

The terminal 100 executing the medical consultation program may perform the medical consultation support method. To this end, the terminal 100 may perform a medical information display function, a medical-consultation-related content display function, a graphical user interface applying function necessary for the production of a medical consultation content, a record receipt function, a verification function, and an information transmission function. In view of this point, the terminal 100 may be configured with functional components, such as an information display module 201, an information input module 202, a data verification module 203, and a transmission module 204 and an insurance verification module 205. The insurance verification module 205 may be considered to be included in the processor 130.

If the medical consultation program is executed, the functional components including the information display module 201, the information input module 202, the data verification module 203, the transmission module 204 and the insurance verification module 205 drive the above-mentioned real components of the terminal 10 in order to perform the medical information display function, the record receipt function, the verification function and the information transmission function.

In detail, the information display module 201 may enable one of general member registration content, specialized member registration content, medical institution registration content, doctor registration content, pharmaceutical company registration content and insurance company registration content to be displayed through the display unit 140. At this time, the user may input a previously set range of information through the input/output module which is driven by the information input module 202. The input information may be transferred to the operating server 300 by the transmission module 204 and stored in a classification of the database corresponding to the character of the user. In accordance therewith, the user may be registered to the medical consultation program.

A registered member may access the medical consultation program through a login procedure. In this case, the information display module 201 may force member login content stored in the member management database to be displayed through the display unit140. At this time, the registered member may input a previously set range of information through the input/output module which is driven by the information input module 202. The input information may be transferred to the operating server 300 by the transmission module 204. The operating server 300 may verify the transferred information on the basis of the previously stored registration information within the member management database, determine whether or not permitting the access to the medical consultation program, and transmit a previously set range of medical information corresponding to the previously stored registration information.

The above-mentioned several registration contents may be read from the member management database by the terminal 100. The registration information used for the member registration may be transferred from the terminal 100 and stored in the member management database. Also, the registration information may be used to determine the range of the medical information which will be transmitted to and received from the operating server 300.

Such a medical consultation program may allow the medical consultation support method to be performed in different modes from one another in accordance with the character of a user. A specialized member login mode being logged-in by a specialized member becoming the consultant, a general member login mode being logged-in by a general member becoming the consulter, a medical institution login mode being logged-in by a medical institution, a pharmaceutical company login mode being logged-in by a pharmaceutical company and an insurance company login mode being logged-in by an insurance company will now be described in detail.

### <Specialized Member Login Mode>

If a specialized member logs-in the medical consultation program, the information display module 201 may enable a previously set range of a menu to be displayed on the display unit 140.

The menu may include icons capable of be selected by a user through the input/output module.

In detail, the menu may include major object name icons and miner object name icons which are classified along with the scale of a consultation object. The consultation object may become a specified region of a body in which a disease is generated or an abnormal disorder symptom due to the disease of a different specified region is revealed, or a cosmetic surgery will be performed for a purpose of beauty and so on. Each of the major object name icons may indicate a large (or wide) region of the body, and the miner object name icons may indicate detailed (or fine or small) portions within the large (or wide) region of the body indicated the major object name icon. As such, each of the major object name icons may be assigned (or allotted) with a plurality of miner object name icons. Such a menu is not limited to this. In another manner, the menu may include major organ name icons indicating external organs of the body and miner organ name icons indicating fine (or small) portions within each of the external organs. Each of the major organ name icon may be assigned (or allotted) with a plurality of miner organ name icons.

When one of the major object name icons is selected through the input/output module, the plurality of miner object name icons may be displayed an adjacent region to the selected major object name icon.

Also, the menu may include major medicine name icons and miner medicine name icons assigned (or allotted) to each of the major medicine name icons. The major medicine name icons may indicate medicine kinds which classifies medicines related various diseases and medicines necessary to perform the cosmetic surgery for beauty and so on based on kinds and/or purposes of the medicines. The miner medicine name icons may indicate each of various medicines.

In other words, the miner medicine name icons may directly indicate the medicine necessary to treat the various diseases and to perform the cosmetic surgery for beauty and so on, and the major medicine name icons may indicate the classified medicine kinds or groups.

The specialized member may select one of plural icons on the basis of the consultation object, such as a disease of a patient, a disorder portion of the body, a region of the body requiring a medical treatment, a cosmetic surgery portion for beauty or others. At this time, an image linked with the selected icon may be read from the memory 120 and displayed on the display unit 140.

The image may become an image stored in the organ database. As such, the image may include information related to one of the external and internal organs and linked with one of the icons.

If the specialized member selects one of the plural icons on the basis of the information about the disease or health of a patient corresponding to a consultation object, the image linked with the selected icon may be read from the organ database and displayed on the display unit 140.

Also, the image may become another image stored in the medicine prescription database. As such, the image may include information which is related to a medicine and prescribing method and action method thereof and linked with one of the icons.

On the other hand, in consideration of variety and complexity in diseases and organs of the body, medical subject name icons capable of classifiedly indicating specialized medical subjects of the specialized members may be included in the menu. For example, if it is possible for a specialized member to handle medical treatments related to at least one of a general internal medicine division, a neurology division, a psychiatry division, a general surgery division, an orthopedics division, a neurological surgery division, a chest surgery division, a plastic surgery division, an anesthesia division, an obstetrics and gynecology division, a pediatrics division, an ophthalmology division, an otorhinolaryngology division, a dermatology division, an urology division, a diagnostic radiology division, a radiation oncology division, an anatomical pathology division and so on, only the medical subject name icons indicating the treatable medical subjects may be selected on the basis of the registration information of the specialized member and the selected medical subject name icons are included in the menu.

The information input module 202 may enable information input from the user through the input/output module of the terminal 100 to be written on the displayed image in a variety of shapes. To this end, the information input module 202 may provide a variety of writing tools on the displayed image in icon shapes. The user may select one of the writing tool icons and write the information on the displayed image using the selected icon. In this case, the user may write the information on the image by touching a touch screen of the terminal.

Also, the information input module 202 may provide recorder icons, which are linked with a voice recording function and an image recording function within the terminal 100 and execute the audio recording function and the image recording function, on the displayed image in the exception of the writing tool icons. As such, the user may select at least one the recorder icons and record at least one of voice and image. The recorded voice and/or image may be stored in the memory 120, and link information about the voice and/or image stored in the memory 120 may be inserted into the displayed image.

The information is inserted in the displayed image in order to produce (or create) a consultation image (or a processed image). The consultation image inserted with the information may be stored in the individual database of the terminal 100 in which an original (or a raw) image is being stored. Also, the consultation image stored in the individual database with the terminal 100 may be transferred to the operating server 300.

As described above, a background (or raw) image and clinical material of each medicine which are necessary for a medical examination and treatment can be provided in accordance with the specialized medical subject at the consultation. As such, the consultant can ease-understandably transfer specialized medical knowledge to the consulter.

The verification module 203 may read verification content from the signature information database and display the read verification content on the display unit 140. The verification content may be used to receive verification information from the user, which corresponds to the consultant, and the consulter which has a medical-related consultation with the user, through the input/output module. The verification information may become handwritten signatures of the consultant and the consulter.

The handwritten signature of the consultant may be stored in a specialized member signature database. The handwritten signature of the consulter may be stored a general member signature database.

The verification content provided by the verification module 203 can include an entire verification icon and a partial verification icon. If at least one of the consultant and the consulter selects the entire verification icon, the handwritten signature written in the verification content may be automatically inserted in all the images which are opened during the consultation. On the other hand, when the partial verification icon is selected, the handwritten signature may be automatically inserted only in previously designated images among all the images which are opened during the consultation.

As described above, not only the handwritten signatures of the consultant and the consulter (that is, a doctor and a patient) but also the handwritten signature of a patient's carer can be inserted in the opened images. As such, reliability of the content used for the medical consultation can enhance. Furthermore, reliability of the medical consultation, examination and treatment can increase.

The verification content can also enable the medical consultation content to be used as a medical treatment confirmation document between the consultant and the consulter. For example, the verification content can allow the medical consultation content to be provided as public and/or private documentary evidence which will be submitted to an insurance company for an application of an enrolled insurance of the consulter. Also, the medical consultation support method can force the terminal 100 of the consultant to receive medical treatment and surgery consent forms for an inpatient and an outpatient from a server within a hospital of the consultant in communication with the hospital server. As such, the consultant can explain a variety of consent forms, which is received from the hospital server and displayed in the terminal 100, to the consulter. As needed, the consultant can explain the variety of consent forms to a carer of the consulter. In this case, a selection for one of the entire and partial verification icons provided by the verification module 203 can force the handwritten signatures on the verification content may be automatically inserted in all or a part of several content, which are used in the consultation between the consultant and the consulter, and the variety of consent forms as the signatures of the consultant and the consulter or the consulter's carer. As such, an additional signing process for the variety of consent forms can be included in the medical consultation procedure. Therefore, the procedure necessary for the following medical treatment procedure or surgery can be simplified.

At a selection of a medical treatment method or a medicine, the handwritten signatures can be selectively inserted in the several content or images, which are opened by the consultant and/or the consulter, using the entire and partial verification icons. As such, a part of several content (or some content) cannot include the handwritten signatures by reason of a non-selection of least one of the medical treatment method, medicines and a medicine appliance, which are necessary for the medical treatment, and so on. In other words, signed content and non-signed content can be distinguished from each other by the handwritten signatures. Therefore, the medical consultation support method and system can increase reliability of the medical consultation content using the signature content.

The transmission module 204 may transmit the above-mentioned consultation images in message shapes which are handled by a social network, a mail and a mobile terminal 100 which are related to at least one of the consultant and the consulter. The transmission module 204 may also transmit the consultation images to an additional server which provides a personal health record cloud service to each of the patients. Moreover, the transmission module 204 may transfer the consultation images to a printer and enable the consultation images to be printed out through the printer.

The consultation images are shared by many people through a text message, the email, the personal health record cloud and the social network. In other words, the patient can share one's own medical treatment information with the other persons. As such, the patient can check one's own medical consultation information through the personal health record cloud at all times. Also, the patient can obtain additional medical information, which reinforces primary medical information forming the basic of one's own medical consultation, from acquaintances or different persons on the social network.

The consulter can receive printed-out consultation image sheets. As such, the consulter can remember the consultation substance (or content) for a long time. Also, the consulter can frequently check the information transferred from the consultant and easily remember the use and precautions of a medicine appliance.

The insurance verification module 205 may receive enrolled insurance information of the consulter from the insurance information presentation server 600 using personal data of the consulter. On the basis of the received insurance information of the consulter, the insurance verification module 205 may determine applicability of the enrolled insurance for a disease and/or medicines described in a currently opened image . Also, the insurance verification module 205 may display the determined resultant on the display unit 140. In accordance therewith, when a medical treatment method and/or a medicine for a disease and so on are selected, the consulter can confirm the applicability of one's own enrolled insurance for the selected medical treatment method and/or medicine. The insurance may include a health insurance operated by a public (or national) institution and various insurances operated by private institutions. As such, the insurance information presentation server 600 may be configured with a plurality of insurance information presentation sub-servers in accordance with the kinds and number of insurance companies.

In order to receive the insurance information using the personal data of the consulter, the terminal 100 may receive and transmit the data from and to the insurance information presentation server 600 via the operating server 300. In this case, the operating server 300 may perform an interface function.

The insurance verification module 205 can allow the consulter to directly check (or confirm) applicability of one's own enrolled insurance for various medical treatment methods, medicines and/or medicine-injection-related appliances, which are cited during the consultation and capable of being used for a disease treatment. As such, the medical consultant procedure using the medical consultation program can substantially help the consulter in an economic aspect (or side).

### <General Member Login Mode>

The general member may download and install the medical consultation program in one's own terminal 100 and may register oneself to the operating server 300 using the medical consultation program. Thereafter, the general member may receive and read one's own medical consultation record from the operating server 300. At the registration of a general member, registration information of the general member may be stored in the operating server 300. If the general member logs-in, the operating server 300 may determine whether or not permission of the log-in on the basis of the registration information of the general member.

In this case, the information input module 202 may receive log-in information from the general member, and the information display module 201 may display a consultation record read icon capable of being selected by general member. If the consultation record read icon is selected by the general member, the information input module 202 may receive the medical consultation record of the general member from the operating server, and the information display module 201 may not only display the received medical consultation record on the display unit 140 but also auditorily output the received medical consultation record through the input/output module.

In this manner, the medical consultation support method according to an embodiment of the present disclosure can allow an easy access of the general member to the medical consultation record. As such, the general member can frequently check (or confirm) one's own medical consultation details through one's own terminal 100.

Also, without any additional medical consultation program only for the general member, the general member easily access the medical consultation information in such a manner as to check (or confirm) information stored in the personal health record cloud. As such, inconveniences caused by download and installation of a program can be minimized, and the medical consultation information of a private person can be quickly checked (or confirmed) through the personal health record cloud.

### <Medical Institution Login Mode>

The medical institution may download and install the medical consultation program in one's own terminal 100 and may register oneself to the operating server 300 using the medical consultation program. Thereafter, the medical institution may receive and read at least a part of medical consultation details of doctors and so on which belong the medical institution, from the operating server 300.

At the registration of a medical institution, registration information of the medical institution may be stored in the operating server 300. If the medical institution logs-in, the operating server 300 may determine whether or not permission of the log-in on the basis of the registration information of the medical institution.

In this case, the information input module 202 may input log-in information from a medical institution member and receive information about doctors, which belong to the medical institution, from the operating server 300. The information display module 201 may display names of the doctors belonging to the medical institution in icon shapes. Such doctor name icons may be choicely selected by the medical institution member . If one of the doctor name icons is selected, the information input module 202 may receive the medical consultation record of a doctor, which belongs to the medical institution and corresponds to the selected icon, from the operating server 300. Also, the information display module 201 may display the received medical consultation record on the display unit 140 and auditorily output the received medical consultation record through the input/output module.

In this manner, the medical consultation support method according to an embodiment of the present disclosure can allow an easy access of the medical institution, which employs the doctors becoming the consultants, to the medical consultation record. As such, the medical institution can frequently check (or confirm) the medical consultation details about each of the employed consultants. Furthermore, the medial institution can unifiedly check (or confirm) and manage consulting methods and times of the employed consultants and the present state of usage of the medical consultation support method.

### <Pharmaceutical Company Login Mode>

The pharmaceutical company may download and install the medical consultation program in one's own purchased terminal 100 and may register oneself to the operating server 300 using the medical consultation program. Thereafter, if the pharmaceutical company may logs-in to the medical consultation program using the registration information, the information display module 201 may display icons, which are necessary to perform a medicine registration procedure. The displayed icons may be choicely selected by the pharmaceutical company. Also, the information input module 202 may receive a content, which corresponds to the selected icon, from the operating server 300. The information display module 201 may display the received content. At this time, the pharmaceutical company can input general medicine-related information, which includes names, prices, pharmacological mechanism, directions, purposes and effects of one's own deal medicines, through the input/output module. The input information may be transmitted to the operating server 300.

Also, the pharmaceutical company can receive and check (or confirm) information, which is related to not only the present state of usage of the content provided by one' own medicinal information presentation server 500 but also sale and use degrees of medicines and so on written on the content, from the operating server 300.

### <Insurance Company Login Mode>

The insurance company may download and install the medical consultation program in one's own terminal 100 and may register oneself to the operating server 300 using the medical consultation program. Also, the information input module 202 may input a unique identification number from the insurance company and receive a list about the consulters, which agrees to provide insurance claim information to the insurance company, from the operating server 300 using the input unique identification number. At this time, the information display module 201 may display the received list. As such, the information input module 202 may input selection data about at least one consulter selected among the consulters on the displayed list and receive at least a part of the medical consultation details for the selected consulter from the operating server 300 using the input selection data. The received medical consultation details of the selected consulter may be displayed by the information display module 201. In accordance therewith, the insurance company can check (or confirm) the medical consultation details and signatures of the consulter and the consultant. Accordingly, the insurance company can entirely manage and verify insurance programs applied to the consulters, payment of insurance benefit, receipt of insurance premium and so on.

### <Detailed Description for Medical Consultation Support Method of Embodiment>

Figure 9 is a flow chart illustrating step by step a medical consultation support method according to an embodiment of the present disclosure. Figure 10 is a photograph showing an example of a log-in page which is provided by a medical consultation program. Figure 11 is a graphical diagram illustrating a function of automatically listing up information about consulters.

Figure 12 is a detailed flow chart illustrating a medical consultation content generation procedure in Figure 9. Figure 13 is a photograph showing an example of a content selection page which is necessary to draft a disease diagnosis content. Figure 14 is a photograph showing an example of a drafting page of disease diagnosis content. Figure 15 is a photograph showing an example of a drafted disease diagnosis content. Figure 16 is a photograph showing another example of a drafting page of disease diagnosis content. Figure 17 is a photograph showing an example of a drafting page of medical treatment method consultation content.

Figure 18 is a photograph showing an example of a content selection page necessary to draft medicine consultation content. Figure 19 through 23 are photographs each showing examples of drafting pages of medicinal consultation content. Figure 24 is a photograph showing an example of a drafting page of cost description content. Figure 25 is a photograph showing an example of a drafting page of signature content which will be used for the verification of a medical consultation. Figure 26 is a photograph showing an example of a drafting page of survey content for post marketing surveillance. Figure 27 is a photograph showing an example of a printable medical consultation content derived from a medical consultation content.

The medical consultation support method according to an embodiment of the present disclosure will now be described in detail with reference to Figure 9 through 27. The medical consultation support method will be performed by the terminal 100 of a consultant which executes the medical consultation program.
1. The terminal 100 may start the performance of the medical consultation support method when the consultant logs-in (S101) .

In detail, the terminal 100 may input log-in information from the consultant and verify the input log-in information using previously stored verification information. If the input log-in information is the same as the verification information, the terminal 100 may permit an access of the consultant to oneself and start the performance of the medical consultation support method for a medical consultation with a consulter.

For example, a specialized member which is registered in the operating server 300 and corresponds to a consultant can input the log-in information on a log-in page which is displayed in the terminal 100 as shown in Figure 10. At this time, the processor 130 can verify whether or not the input log-in information is the same as registration information previously stored in the specialized member management database12 of the memory 120.

In another manner, the input log-in information can be transferred to the operating server 300 through the communication unit 110. As such, the operating server 300 can verify whether or not the input log-in information matches the registration information previously stored in one's own database. The terminal 100 can finally determine whether or not a permission of the log-in of the specialized member on the basis of a verification resultant received from the operating server 300.
2. And then the medical consultation support method can be performed. As such, the terminal 100 may display a list about consulters which will consult the consultant (S102).

In detail, the terminal 100 of the consultant may receive information about the consulters from at least one of a terminal 100 of a nurse, an electronic health record system (EHR) of the terminal 100 of a consulter and an electronic medical record system (EMR) of a respective hospital and so on through one's own communication unit 110. Also, the terminal 100 of the consultant may display the received consulter information on the display unit 140 in a list shape.

For example, the terminal 100 of the consultant can receive the list about the consulters, which will receive medical examination and treatment today, from the electronic medical record system of the respective hospital. The consulters on the list can be arranged along with a consultation sequence.
3. Also, the terminal 100 of the consultant can sense (or detect) a consulter staying within a previous set distance (S103).

In detail, the terminal 100 of the consultant can check the position of one of a terminal 100 and a wearable device of the consulter, in which the medical consultation program is installed, through one's own communication unit 110 and determine whether or not the consulter is positioned within a previously set distance.

In other words, the terminal 100 of the consultant can sense the position of the consulter and check whether or not a hospital arrival of the consulter and information of the consulter entered in a medical room.

Also, the terminal 100 of the consultant can display the consulter on the list in one of activation and non-activation states according whether or not the hospital arrival of the consulter (S104 and S105).

For example, if a second terminal 100 of a second consulter P2 enters within a second distance d2 which is previously set on the basis of the size of a hospital, the terminal 100 of the second consulter P2 can transmit one's own position information to the operating server 300 and/or the terminal 100 of the consultant. As such, the terminal 100 of the consultant can sense the position of the second consulter P2 and confirm a hospital arrival of the second consulter P2. When the hospital arrival of the second consulter P2 is confirmed, the terminal 100 of the consultant displays the second consulter P2 on the list in the activation state and informs the consultant of the hospital arrival of the second consulter P2 (S105).

Also, when the terminal 100 of a first consulter P1 enters within a first distance d1 which is previously set on the basis of the size of a medical room, the terminal 100 of the first consulter P1 transmits one's own position information to the terminal 100 of the consultant and/or the operating server 300. As such, the terminal 100 of the consultant can receive the position information about the first consulter's terminal 100 and sense the position of the first consulter. The terminal 100 of the consultant can identify a consulter entered into the medical room with the first consulter. At this time, the terminal 100 of the consultant can emphasize the first consulter P1 on the list in one of pop-up and highlight states and recommend the consultant to select the execution of a medical consultation support function (or method) for the first consulter P1.

In this manner, the terminal 100 of the consultant can receive the position information from the terminal 100 of the consulter and confirm (or check) whether or not the hospital arrival of the consulter and the entrance of the consulter to the medical room on the basis of the received position information. As such, the terminal 100 of the consultant can help the consultant to easily find the information about the consulter, which will receive the medical examination and treatment, and enable the medical consultation support method for the consulter to be directly executed.

The executed medical consultation support method can enable the electronic medical record, which includes blood-sugar level, weight, blood pressure and so on of the consulter and is necessary to either diagnose a disease of the consulter or check a progress state of the disease of the consulter, to be displayed on the display unit 140 of the consultant's terminal 100. As such, the medical consultation support method can support (or assist) a diagnosis procedure of the consultant.

The continuously measured electronic medical record of the consulter can be displayed on the display unit 140 in an easily recognizable image shape including a graph and so on. Moreover, additional information either estimating a disease of the consulter or representing the progress state of the disease in numerical data can be displayed on the display unit 140. The additional information can be obtained by analyzing the electronic medical record of the consulter using an artificial intelligence-based big data analysis technology.
4. Thereafter, the terminal 100 can apply the graphical user interface which is used to draft (or create) the medical consultation content (S106).

In detail, the terminal 100 can provide a medical consultation-related content search window (or a search box) . As such, the consultant can easily select (or find) the medical consultation-related content supporting (or assisting) the medical examination and treatment. The terminal 100 can display the medical consultation-related content selected by the consultant.

The selected medical consultation-related content can be printed-out by being transferred from the terminal 100 to a printer, and provided to the consulter as a visual image material at the medical consultation. In accordance therewith, the quality of the medical consultation can be enhanced.

Also, the terminal 100 can apply the graphical user interface necessary for the consultant to input written data, voices and/or images. As such, the medical consultation content including the input of the consultant can be created (or generated).

In order to effectively receive the input of the consultant (or user) while the visual images supporting (or assisting) the medical consultation are displayed, it is preferable to provide the graphical user interface using a touch screen which unites the input unit 150 and the display unit 140 with each other. Therefore, the display unit 140 and the input unit 150 cited in the following description can be considered to designate the touch screen.

A generating procedure of the medical consultation content according to an embodiment of the present disclosure will now be described in detail.

As described above, the medical consultation content can include at least one of a disease diagnosis content, a treatment method content, a medicinal consultation content, a medical treatment cost content, an insurance information content, a signature content and any other content.

In this disclosure, the medical consultation content can become a serial which unifies the disease diagnosis content, the treatment method content and the medicinal consultation content in a consecutive sequence.
4-1. In order to draft (or create or generate) the medical consultation content, the terminal 100 can display a content search and selection page for searching content necessary to draft (or create or generate) the disease diagnosis content (S201).

The disease diagnosis content can include visual images which will be used to explain a disease of the consulter. For example, the disease diagnosis content can include information at least one of a body organ image, a definition of the disease, symptoms of the disease, trouble sources of the disease, and diagnosis or examination procedure of the disease.

In other words, the terminal 100 can display a disease diagnosis content search/selection page. As such, the consultant can search and select any disease diagnosis content necessary to explain a disease of the consulter.

In this disclosure, the disease diagnosis content search/selection page can include a disease menu tree 2011 specifying diseases of the consulters as shown in Figure 13. The disease menu tree 2011 on the disease diagnosis content search/selection page can implemented in an icon system of at least two stages (or steps) . For example, the disease diagnosis content search/selection page can be configured with one of an object name icon set and an organ name icon set. The object name icon set can include major object name icons and minor object name icon, and the organ icon name set can include major organ name icons and minor organ name icons. As such, the consultant (or user) can sequentially select one of the major icons and one of minor icons belonging in the selected major icon, and easily search (or find) a proper disease diagnosis content suitable to explain the disease of a consulter.

In the disease diagnosis content search/selection page, the menu tree can enable not only the major organ name icons to be arranged in a higher rank but also the minor organ name icons belonging in each of the major organ name icons to be arranged in a lower rank. As such, the disease diagnosis content search/selection page can include a filtered menu tree corresponding to the medical treatment subjects of the specialized member. The filtered menu tree can include some major name icons corresponding to the medical treatment subjects of the specialized member and the minor name icons belonging in some major name icons. Such a filtered menu tree can be obtained by filtering the major and minor name icons on the basis of the registration information of the specialized member.

If the consultant is an internist, a filtered menu tree configured with higher content icons corresponding to intern-related diseases, which are frequently handled by the consultant and include an irritable bowel syndrome, a rheumatoid arthritis and so on, and lower content icons corresponding to detailed diseases, which pertain to each of the intern diseases, can be displayed on the disease diagnosis content search/selection page, as shown in Figure 13.

For example, if the consulter is a patient with spondylolysis, the consultant can sequentially select a waist (or a lumbar vertebra) icon corresponding to a major name icon, a spine icon corresponding to a minor name icon, and the spondylolysis pertaining to a spine-related disease.

When the spondylolysis is selected, the terminal 100 can read visual images related to the spondylolysis from the memory 120 or the database of the operating server 300, draw up a list about the read visual images, and display the prepared image list. As such, visual images suitable to draft (or create or generate) the disease diagnosis content can be selected among the read visual images by the consultant.

Actually, the visual images may include texts and/or images about spine-related body organ, disease definition, disease symptoms, disease source, and diagnosis and examination procedures. Such visual images may be sequentially listed-up by the terminal 100 on the basis of their own inquiry number, score (or grade), reliability and use history. As such, the consultant can select at least one of the listed-up visual images, and so the terminal 100 can display the selected visual image on a drafting page of the disease diagnosis content (or can insert the selected visual image into the disease diagnosis content being drafted).

In a different manner, the terminal 100 can list up medical consultation content serials, which are previously drafted for the spondylolysis by the consultant or another person, and display the prepared list. Also, the terminal 100 can sequentially output (or display) several disease diagnosis contents selected by the consultant.

In another different manner, the terminal 100 can directly input a disease name from the consultant and search a disease diagnosis-related content corresponding to the input disease name.

If an image suitable to draft (or create or generate) the disease diagnosis content is selected, the terminal 100 can display the selected image on a screen of the display unit 140 and receive an input of the consultant (S202).

In detail, the terminal 100 can display the selected image on the display unit 140 and receive the input of the consultant, such as written data, voices, edited image and so on, through the input unit 150. Also, the terminal 100 can insert into the displayed visual image. In accordance therewith, a new disease diagnosis content with an edited visual image can be generated (or created).

In the drafting page of the disease diagnosis content, a body image related to a disease to be diagnosed can be displayed (or inserted). Also, names for detailed portions within the displayed body image, disease name, disease definition and so on can be additionally inserted.

To this end, the processor 130 can control not only image information, which is previously stored in the memory 120 corresponds to the icon selection of the consultant, to be read but also the display unit 140 to display the read image information. In another manner, the processor 130 can control not only the communication unit 110 to receive image information, which is previously in the organ database of the operating server 300 and corresponds to the icon selection of the consultant, from the operation server 300 but also the display unit 140 to display the received image information.

For example, if the consulter is a patient with the spondylolysis, a spine image 2121 of a different patient with the spondylolysis similar to the state of the consulter can be displayed in a drafting page of the disease diagnosis content, as shown in Figure 14. Also, character data and graphical data related to a disease position 2131, detailed organ names 2141, a disease source indicator 2151 and a disease name 2161 can be additionally inserted in the drafting page of the disease diagnosis content as disease information about the spondylolysis. Moreover, a spine image 2111 of a normal person without the spondylolysis can be additionally displayed in the drafting page of the disease diagnosis content, in order to effectively assist in a disease explanation. Furthermore, an animation or images representing a procedure which progresses (or varies) from a normal spine image to an abnormal spine image with the spondylolysis in process of time can be displayed in the drafting page of the disease diagnosis content.

Continuously, the terminal 100 can receive the input of the consultant (or the user) through the input unit 150, the camera unit 170 and so on and combine the input received from the consultant (or user) with the displayed visual image. In accordance therewith, the terminal 100 can generate (or create) the disease diagnosis content explaining the disease of the consulter.

Actually, as shown in Figure 15, the consultant can handwrite data, such as characters and graphic signs, on the visual image displayed in the drafting page of the disease diagnosis content during a consultation with the consulter. In this case, the input unit 150 can sense the written data of the consultant and the enable the handwritten data 2501 to be displayed in the display unit 140. The handwritten data 2501 can be combined with the visual image displayed in the drafting page of the disease diagnosis content. Accordingly, the disease diagnosis content can be generated (or created).

Also, the terminal 100 can record a conversation of the consultant with the consulter using the input unit 150 can combine the recorded conversation of the consultant and the consulter with the visual image displayed in the drafting page of the disease diagnosis content, in order to generate (or create) the disease diagnosis content.

Moreover, the terminal 100 can photograph a consultation scene of the consultant and the consulter through the camera unit 170 and combine the photographed images with the visual image displayed in the drafting page of the disease diagnosis content, in order to generate (or create) the disease diagnosis content. The camera unit 170 can be installed at an external position separated from the terminal 100 and photograph the consultation scene at the external position of the terminal 100. The photographed images can be transferred from the camera unit 170 to the terminal 100 in one of wire and wireless communication modes.

Furthermore, the terminal 100 can provide at least one optional function at a draft of the disease diagnosis content as shown in Figure 16, in order to more easily draft (or generate or create) higher quality disease diagnosis content. The at least one optional function can include a live clinic function 2211, a privacy protection function 2221, a viewing direction change function 2231 and a voice-to-text conversion function 2241.

The optional functions used for the content draft will now be limitedly described as an example of drafting the disease diagnosis content, but they are not limited to this. In other words, the optional functions can be used to draft different medical consultation content.

The live clinic function can sequentially store a writing procedure of the user (or consultant) and combine the stored writing procedure with voices recorded during the writing procedure. In accordance therewith, the live clinic function can generate (create) the medical consultation content in an animation shape.

If the consultant executes the live clinic function, written data being input by the consultant can be sequentially stored from a start time point the execution of the live clinic function in process of time. Also, consultation voices can be sequentially recorded together with the written data in process of time. Moreover, a consultation scene can be recorded in process of time.

If the disease diagnosis content generated using the live clinic function is output, the writing procedure progressing in process of time can be displayed on the display unit 140 in an animation shape as well the recorded consultation voice can also be output through the speaker 180. Such a live clinic function can enable information about the disease consultation to be more affluently and realizably transferred.

The privacy protection function can enable an input data of the user (or consultant and/or consulter) input from a start time point of the execution of the privacy protection function to be not included in the disease diagnosis content. In accordance therewith, the privacy of the consulter can be protected.

For example, if the consultant selects the execution of the privacy protection function, the terminal 100 cannot sense the input data, such as written data, voices, images and so on, from the start time point of the execution of the privacy protection function. In another manner, when the execution of the privacy protection function is selected by the consultant, although the input data of the user input from the start time point of the execution of the privacy protection function is sensed, the terminal 100 can enable the sensed input data to be not included in the disease diagnosis content.

The viewing direction change function can allow a written input data of the consultant to be turned at a previously set angle and displayed in the turned state. This may result from the fact that the consultant and the consulter are generally opposite to each other at the consultation.

In detail, the consultant can write an input data on a drafting page of the disease diagnosis content in a consultant's viewing direction and select the viewing direction change function. At this time, the terminal 100 can turn the written input data at a designated angle and display the written input data in a consulter's viewing direction. For example, as shown in Figure 16, when the consultant writes an input data of ' 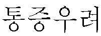 ' 2501 in one's own viewing direction and then selects the viewing direction change function, the written input data of ' 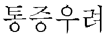 ' 2501 can be turned in the consulter's viewing direction (that is, at an angle of 180 degrees) and displayed in the turned state. Therefore, the consultant can be easy to write an input data in one's own viewing direction, as well the consulter can be easy to watch the written input data in one's own viewing direction.

The voice-to-text conversion function can convert voices recorded during the consultation into texts and insert the converted texts into a visual image displayed on the drafting page of the disease diagnosis content.

The texts converted from the recorded voices can be inserted in a previously designated region 2601 of the visual image displayed on the drafting page of the disease diagnosis content, as shown in Figure 16. Such inserted consultation texts can be included in the disease diagnosis content.

On the other hand, previously drafted disease diagnosis content can be selected and re-displayed (or re-used) . In this case, the previous disease diagnosis content can be provided as a visual image displayed in a drafting page of secondary disease diagnosis content. As such, the consultant can edit the previous disease diagnosis content displayed in the drafting page of the secondary disease diagnosis content by inserting an input data. In accordance therewith, the secondary disease diagnosis content can be generated (or created).

Such disease diagnosis content can include medical treatment method content. As such, the terminal 100 can provide a drafting page of the medical treatment method content.

In the drafting page of the medical treatment method content, a visual image related to a medical treatment method (for example, a surgery method), a prophylaxis, a progress, a complication, food and nutrition information and so on can be displayed.

For example, as shown in Figure 17, a spine image 2211 with a spondylolysis and another spine image 2221 with a spondylolysis surgery can be displayed in the drafting page of the medical treatment method content. Also, a description about the medical treatment method can be additionally displayed in the drafting page of the medical treatment content.

The consultant (or user) can additionally insert one's own comment 2501 in the drafting page of the medical treatment method content and generate (or create) the medical treatment method content.
4-2. After the disease diagnosis content is completed, the terminal 100 can display a content search/selection page for searching medicine content suitable to draft medicinal consultation content (203).

As an example of the content search/selection page, the terminal 100 can display a medicine search page, which is able to directly input a medicine name, in the display unit 140.

In another manner, the display unit 140 of the terminal 100 can display a medicine content selection page including maj or medicine name icons and minor medicine name icons opposite (or pertaining) to each of the major medicine name icons, as shown in Figure 18. For example, an icon arrangement (or a menu tree) 2021 including higher content icons, such as a medicine A, a medicine B and so on, and lower content icons opposite (or pertaining) to each of the higher content icons can be displayed in the medicine content selection page.

In still another manner, the medicine content selection page can include a filtered icon arrangement (or a filtered menu tree) corresponding to the medical treatment subjects of the specialized member. The filtered icon arrangement (or the filtered menu tree) can include some major medicine name icons related to diseases corresponding to the medical treatment subjects of the specialized member and the minor name icons pertaining to some major name icons. Such a filtered icon arrangement (or the filtered menu tree) can be obtained by filtering the major and minor medicine name icons on the basis of the registration information of the specialized member.

In further still another manner, the medicine content selection page can include another filtered icon arrangement (or another filtered menu tree) configured with major and minor medicine name icons which are related to diseases corresponding to selected one of the major and minor object name icons and/or the major and minor organ name icons of the disease diagnosis content search/selection page. The another filtered icon arrangement can be obtained by filtering the entire major and minor medicine name icons on the basis of the object or organ icon selected by the specialized member in the disease diagnosis content selection procedure.

If the selection of the medicine content suitable to draft the medicinal consultation content is completed, the terminal 100 can display the selected medicine content on the screen of the display unit 140 and receive input data of the consultant (S204).

In detail, the processor 130 can control not only a previously stored medicine content corresponding to the medicine name icon selected by the consultant to be read from the memory 120 but also the display unit 140 to display the read medicine content. In another manner, the processor 130 can control not only the communication unit 110 to receive a medicine content, which is stored in the medicinal prescription database 30 and corresponds to the medicine name icon selected by the consultant, from the operating server 300 but also the display unit 140 to display the received medicine content. The medicine content can include images shown in Figures 19 through 23.

More specificationally, the terminal 100 can read a medicine information image 2301 shown in Figure 19 from the medicine information database 31 and display the read medicine information image 2301 in a drafting page of the medicinal consultation content. As such, the specialized member corresponding to the consultant can write a variety of input data on the displayed medicine information image 2301 through the input unit 150.

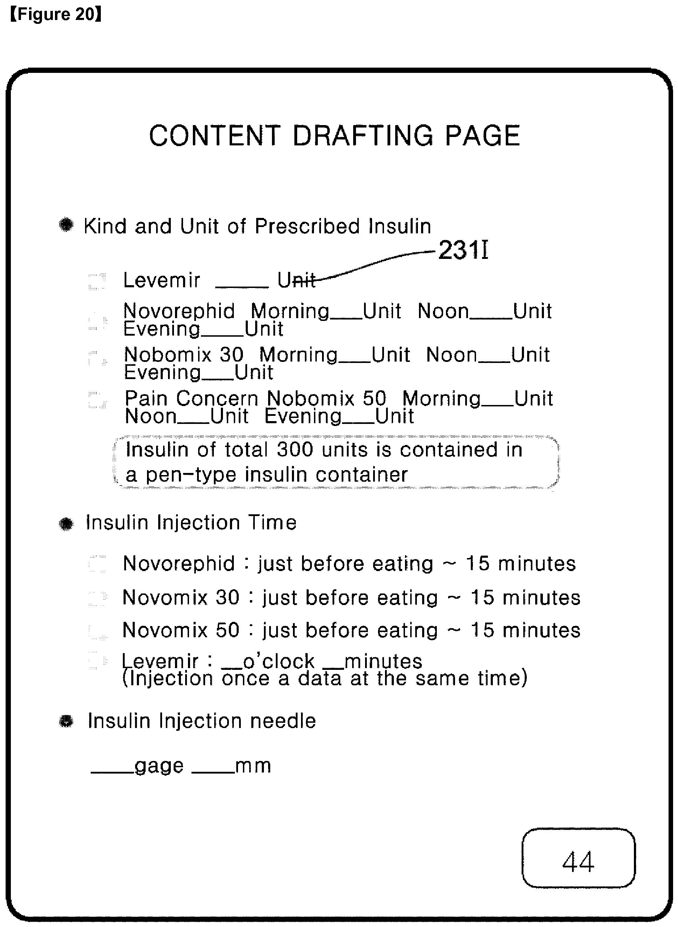

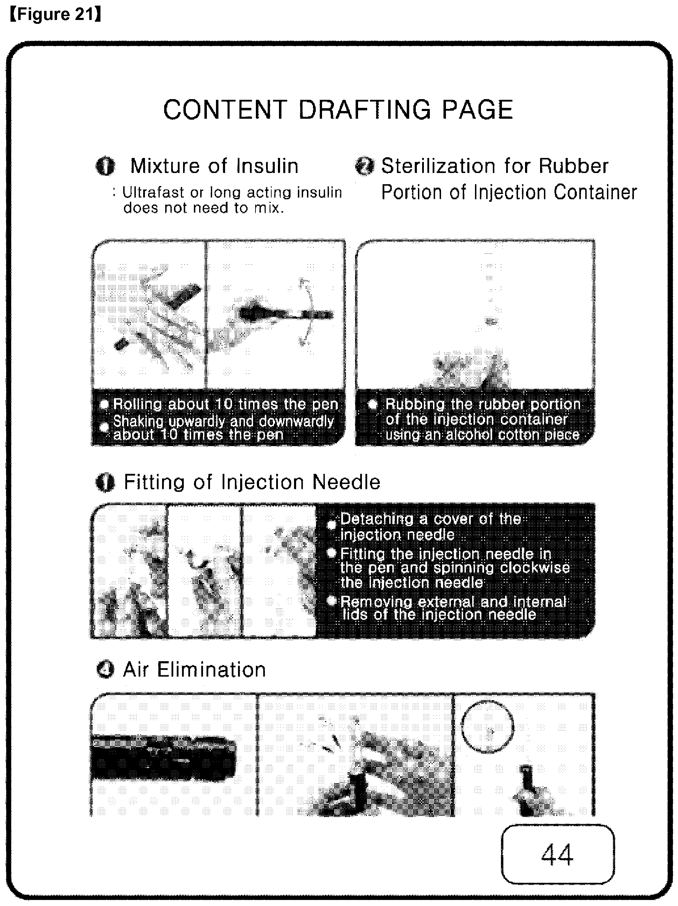

Also, the terminal 100 can sequentially read medicine prescription information images shown in Figures 20 and 21 from the medicine prescribing method database 32 and sequentially display the read medicine prescription information images in one and another different drafting pages of the medicinal consultation content. As such, the consultant can write a variety of input data on the displayed medicine prescription information images.

The medicine prescription must vary along with an individual status of the consulter. One of the medicine prescription information images capable of being used as medicine prescription content can include blanks which will be filled with written prescription data.

The medicine prescription information image shown in Figure 20 can include medicine names, blanks 2311 which will be filled with dosages of the respective medicines, different blanks 2311 which will be filled with dosing time points, and/or another different blank 2311 which will be filled with a dosing appliance, and so on. As such, the consultant can easily write prescription data for the consulter on the medicine prescription information image in consideration of age and disease progressing state of the consulter.

For the convenience of the consultant and a correct prescription, the processor 130 can calculate medicine prescription values on the basis of previously input information about the consulter and a disease diagnosis and automatically insert the calculated medicine prescription values in the blanks 2311 of the medicine prescription information image.

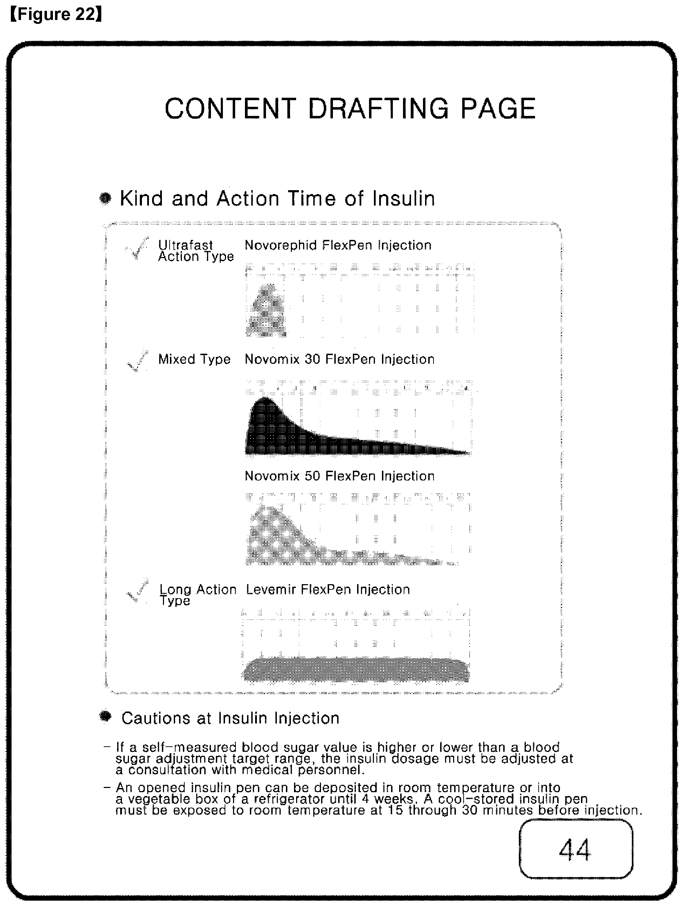

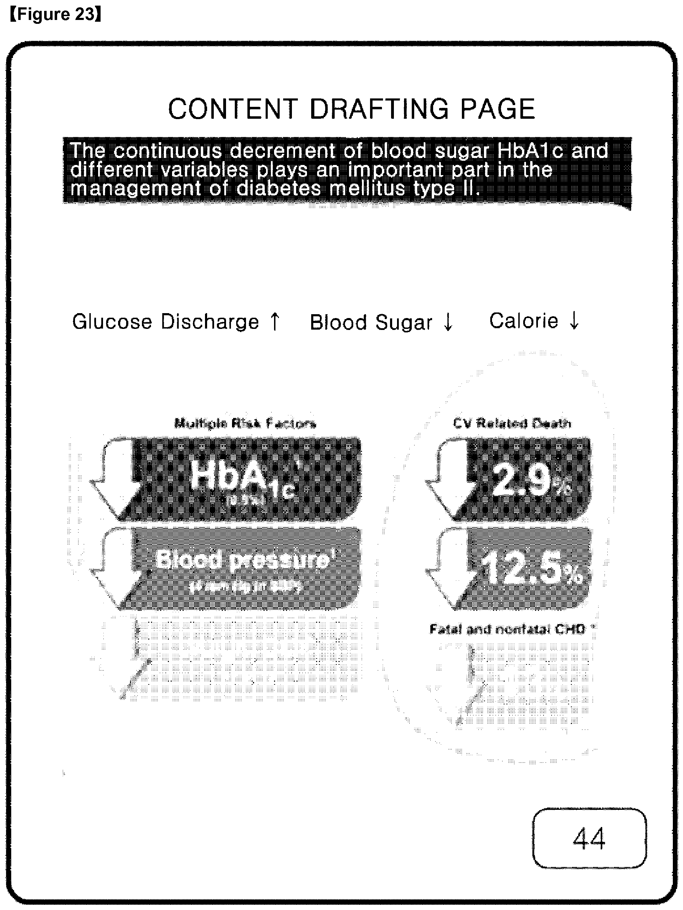

Moreover, the terminal 100 can control a medicine action mechanism information image and a pharmacological mechanism information image shown in Figures 22 and 23 to be read from the medicine action mechanism database 33 and sequentially displayed in still and further still different drafting pages of the medicinal consultation content. As such, the consultant can write a variety of input data on the medicine action and pharmacological mechanism information images.

On the other hand, the display unit 140 can first display the major object name icons, the minor object name icons, the major organ name icons and the minor organ name icons indicating images stored in the organ database 20 as well enable the consultation using the image stored in the organ database 30 to be proceeded. Subsequently, the display unit 140 can display the major medicine name icons and the minor medicine name icons indicating images stored in the medicinal prescription database 30.

The processor 130 can store icons selected by the consultant in the memory 10 and enable the previously selected icon, which is used in the previous consultation and stored in the memory 120, to be displayed on the display unit at first or at the highest position or in a favicon or bookmark shape during next consultation. As such, the consultant can easily select the icons indicating images which are frequently used in accordance with the medical treatment subject of the consultant. Also, the icons indicating the images stored in the organ database 20 can be display in a linked state with the icons indicating the images stored in the medicinal prescription database 30. Therefore, not only the body organ images necessary for the consultation of various diseases can be easily selected, but also the images including information about the medicines and/or a medicine injector, which are necessary for the respective body organ and the consultation of a respective disease, can be easily selected.
4-3. If the draft of the medicinal consultation content is completed, the terminal 100 can display a drafting page of cost description content which is based on the diagnosis information, the medical treatment information and the medicine prescription information and enable input data of the user (or consultant) to be inserted in the drafting page of cost description content (S205 and S206) . As such, the cost description content can be drafted (or generated or created). Such cost description content can include insurance information.

In detail, the cost description content can include information about a diagnosis cost, a medicine cost, a medical treatment (surgery) cost and so on. The cost description content can also include information about applicability of public and/or private insurances for each of the costs.

As such, the drafting page of the cost description content can include information about diagnosis, medical treatment and medicines, which are provided to the consulter. The drafting page of the cost description content can also include information about the applicability of the insurance for each of the diagnosis, medical treatment and medicines.

In order to prepare the drafting page of the cost description content, the terminal 100 can directly retrieve enrolled private insurances of the consulter and received information about the enrolled private insurances of the consulter from the insurance information presentation server 600. Also, the terminal 100 can determine applicability of the enrolled private insurances of the consulter and display the determined applicability of the enrolled private insurances of the consulter, as shown in Figure 24.

In other words, the terminal 100 can receive private insurance enrollment information of the consulter from one of the operating server 300 and the insurance information presentation server 600 using membership information of the consulter. Also, the terminal 100 can determine the applicability of the private insurance on the basis of the received private insurance enrollment information and display the determined applicability of the private insurance.

Applicability of the public insurance can be determined on the basis of corrective public insurance regulations. As such, the processor 130 of the terminal 100 can determine the applicability of the public insurance for the diagnosis, medical treatment and medicines on the basis of stored public insurance regulations and enables the determined resultant to be displayed.
4-4. The cost description content is completed, signature content representing a termination of the medical consultation can be drafted (S207).

Referring to Figure 25, the display unit 140 can display a drafting page of the signature content and enable the consultant and the consulter to handwrite their signatures through the input unit 150.

The drafting page of the signature content can include a signature cancel icon, a partial consent signature icon and an entire consent signature icon. If the signature cancel icon is selected through the input/output module, the handwritten signatures can be deleted. When the partial consent signature icon is selected through the input/output module, the handwritten signatures on the drafting page of the signature content can be automatically inserted only in some images, in which a consent receipt icon is selected, among images of the medical consultation content. If the entire consent signature icon is selected through the input/output module, the handwritten signatures on the drafting page of the signature content can be automatically inserted in all the images of the medical consultation content which are opened and include handwritten data.
4-5. Thereafter, the terminal 100 can perform a procedure of drafting an additional content (S208).

In detail, if the consulter doses oneself with a medicine necessary for a post marketing surveillance (PMS), the terminal 100 can display a drafting page of survey content, which is necessary for the post marketing surveillance (PMS), in the display unit 140. The terminal 100 can receive input data of the consultant and the consulter and generate the survey content for the post marketing surveillance (PMS).

For example, the drafting page of the survey content can include a survey image and signature panels 2711 and 2721 which will be filled with signatures of the consultant and the consulter. As such, the consultant and the consulter can easily draft the survey content for the post marketing surveillance using touch inputs. The terminal 100 can transmit the drafted survey content for the post marketing surveillance PMS to the respective pharmaceutical company server through the communication unit 110. Therefore, the consultant and the consulter can easily respond to the post marketing surveillance PMS.

In this way, when the various content included in the medical consultation content are sequentially drafted (or generated or created), the processor 130 can unify the drafted various content and generate a series of medical consultation content.

In other words, the processor 130 can connect the drafted various content with one another in a drafting sequence and generate the medical consultation content related to a specified disease.

For example, the processor 130 can generate the medical consultation content by aligning and weaving the drafted disease diagnosis content, medical treatment method content and medicinal consultation content in the drafting sequence.
5. The processor 130 can properly transform the generated medical consultation content in accordance with a specified purpose (107).

For example, the processor 130 can transform (or edit) the medical consultation content into a printable medical consultation content, in order to provide the medical consultation content to the consulter in an off line mode.

In order to transform the medical consultation content into the printable medical consultation content, the processor 130 can transform the sequentially input written data into a completely written data. Also, the processor 130 can convert the recorded consultation voices into texts and display the converted texts. If an animation or a motion image is included in the medical consultation content, the processor 130 can capture important images and display the captured images as successive process images.

For example, the printable medical consultation content can be configured with a plurality of images 2411, 2421, 2431 and 2441 which are obtained by editing some images of the animation and sequentially aligning the edited images, a completely written data 2501 and a text 2601 converted from the recorded voice, as shown in Figure 27.

Also, the processor 130 can perform a procedure of transforming the medical consultation content into shareable medical consultation content.

In order to share the medical consultation content with the other persons, the medical consultation content must be transformed in the shareable medical consultation content by being edited privacy information and unnecessary information within the medical consultation content. To this end, the terminal 100 can provide the graphical user interface.

For example, the terminal 100 can respond to an instruction of the user (or consultant) through the graphical user interface and control a specified interval of recorded voices to be muted, the privacy information of the consulter to be deleted, or the unnecessary images within the medical consultation content to be erased. As such, the shareable medical consultation content can be easily generated.

Such shareable medical consultation content can be stored in the memory 120 and displayed at a consultation with a different consulter having the same disease later. Therefore, the consultation with the different consulter can more easily proceed.

Also, the shareable medical consultation content can be uploaded to the operating server 300 through the communication unit 110 and used by the other persons. If the shareable medical consultation content is downloaded by the other persons, the operating server 300 can compensate the consultant for the download.

Moreover, the processor 130 can transform the medical consultation content into survey content for a post marketing surveillance (PMS) . For example, the processor 130 can generate the survey content for the post marketing surveillance (PMS) by including the drafted survey image and the signed signature panels.

Finally, when the medical consultation content is completed, the terminal 100 can provide the consulter with the completed medical consultation content in one of on and off line modes (S108).

In detail, the terminal 100 can respond to a selection of the user (or consultant) and transfer the printable medical consultation content to an external printer through the interface unit 160. As such, the printable medical consultation content can be printed out by the external printer.

Also, the terminal 100 can transmit the medical consultation content to a mobile terminal 100 of the consulter through the communication unit 110.

Moreover, the terminal 100 can transmit the shareable medical consultation content to the insurance information presentation server 600 of an insurance company in which the consulter is enrolled. In another manner, the terminal 100 can transfer the shareable medical consultation content to the operating server 300 and the insurance company can check the drafted consultation details of the consulter through access to the operating server 300.

In this case, the display unit 140 can display applicability of insurance for at least one of medical treatment method, medicines and medicine injection appliance and cost information thereby.

The processor 130 can input consulter's personal data from the consulter through the input/output module and transmit the input personal data of the consulter to the insurance information presentation server 600. In this case, the insurance verification module 205 can receive insurance enrollment information of the consulter from the insurance information presentation server 600. As such, the insurance verification module 205 can not only determine applicability of the insurance for at least one of medical treatment method, medicines and medicine injection appliance on the basis of the received insurance enrollment information of the consulter, but also calculate cost information in accordance with the applicability of the insurance. Also, the insurance verification module 205 can enable the determined resultant about applicability of the insurance and the calculated cost information to be displayed.

As described above, the medical consultation support method and terminal in accordance with embodiments of the present disclosure can promote satisfaction of a consulter, such as a patient, for a medical consultation with a consultant.

In detail, the medical consultation support method and terminal in accordance with embodiments of the present disclosure can promote quality of a medical consultation and understanding of a consulter for the medical consultation by sequentially provide a variety of audiovisual materials, which support (or assist) a medical consultation, in accordance with medical examination and treatment procedures at the medical consultation. In other words, the specialized knowledge and unfamiliar medical terms can be easily explained to patients. As such, the medical consultation support method and terminal can induce the patient to easily understand one's own disease and actively try to overcome one's own disease.

More specificationally, the medical consultation support method and terminal in accordance with embodiments of the present disclosure can generate medical consultation content of a high quality for a consulter by preparing a graphical user interface which inputs audiovisual materials and consultation substances (or contents) of a consultant in various modes.

The medical consultation support method and terminal in accordance with embodiments of the present disclosure can apply generated medical consultation content to a consulter in on/off line modes so that not only the consulter checks one's own medical treatment substances (or contents) and shares the medical treatment content with the other people but also a consultant is motivated to increase the quality of the medical treatment. As such, a consulter can not only receive a printed material including medical consultation substances (or contents), medicine instructions, and the proper use of an unfamiliar medicine injector but also continuously remember one's own medical treatment substances (or contents) . Also, the consulter to have helps from intimate persons by sharing the medical consultation content with the intimate person through a social network. Moreover, the consulter can ascertain at a time not only an expense being caused by the application of a therapeutic (or medical treatment) method, medicines and so on necessary for a medical treatment of diseases but also applicability of insurances therefor, and decide a reasonable application of the therapeutic method, the medicines and so on.

Also, the medical consultation support method and terminal in accordance with embodiments of the present disclosure can allow a consultant, such as a doctor, to explain specialized medical knowledge to a consulter using a variety of content. As such, a circumstance of medical examination and treatment can be improved and satisfaction of a patient can be increased. Therefore, it can be a great help for the consultant to attract patients. Also, necessary information, such as the specialized medical knowledge and so on, can be more easily and efficiently transferred to the patient.

Moreover, the medical consultation support method and terminal in accordance with embodiments of the present disclosure can allow pharmaceutical companies to unaffectedly publicize information about directions and effects of their own medicines and so on through an opinion exchange between a consultant and a consulter without simply publicizing their medicines and medicine injection appliances. As such, the medical consultation support method and terminal can actively guide pharmaceutical companies to create a variety of content related to medicine development and pharmaceutical information. Therefore, benefits of the pharmaceutical companies can be increased and a medical consultation circumstance between the consultant and the consulter can be enhanced.

Furthermore, the medical consultation support method and terminal in accordance with embodiments of the present disclosure can previously prevent illegal and unreasonable insurance application and insurance benefit fixing by enabling an insurance company to check consultation details between a consulter and a consultant being a medical specialist and signatures of the consulter and the consultant. Such medical consultation details can be used in development of insurance produce except that they are used as basic materials for the insurance application and the insurance benefit calculation. As such, the medical consultation support method and terminal can enable insurance policies and products, which promote public and private benefits of the pharmaceutical companies and improve a circumstance of the medical consultation between the consultant and the consulter, to be actively developed.

### <Operation Procedure of Operating Server>

Figure 28 is a flow chart illustrating an operation procedure of an operating server which performs a medical consultation support method.

The operating server 300 can receive the medical consultation-related content from the terminals 100 and various servers (S301).

Specificationally, the operating server 300 can receive a variety of visual images, visual and auditory materials and medical consultation content from the terminals 100 and external servers.

The operating server 300 can limit content to be uploaded in accordance with the character of an upload principal-agent. For example, the operating server 300 can allow pharmaceutical companies to upload only medicine-related content, insurance companies to upload only insurance information-related content, and medical institutions to upload only medical consultation content.

If the received content includes a visual image and a visual and auditory material which are used to draft medical consultation content, the operating server 300 does not set any limitation. On the contrary, when the medical consultation content drafted by the consultant is received, the operating server 300 can perform several verification procedures (S302) .

In detail, the operating server 300 can evaluate the reliability of the received medical consultation content. To this end, the operating server 300 can be established to allow only the registration of the received medical consultation content obtaining at least a previously set score from primarily verified specialists.

If the received content is a secondary medical consultation content which is drafted using content uploaded by different person, the operating server 300 can calculate a contribution ratio between the secondary drafter and the different person in the draft of the secondary medical consultation content (S304).

When the verification procedures are terminated, the operating server 300 can classify the received medical consultation-related content in accordance with relevant diseases and patient's features, such as age, sex, race, constitution and so on, and store the classified medical consultation-related content in the database (S305).

Continuously, the operating server 300 can retrieve the variety of medical consultation-related content stored in the database and list up the retrieved various content. Also, the operating server 300 can transfer the listed up information (S306).

For example, the operating server 300 can provide thumbnails, which represent and are aligned in a list shape and represent the various content, to the terminals 100.

Subsequently, the operating server 300 can transfer the medical consultation-related content requested by the terminal 100 (S307).

Finally, the operating server 300 can compensate registrants of medical consultation-related content in accordance with the number of transmission times of the respective medical consultation-related content (S308). Therefore, the operating server 300 can motivate the registrants to actively register the medical consultation-related content.

The above-mentioned medical consultation support method in accordance with an embodiment of the present disclosure can be implemented in a program command shape capable of being executed through various computer components and so stored in a computer-readable record medium. The computer-readable record medium can individually or combinatively store program commands, data files, data structures and so on. A part of program commands stored in the computer-readable record medium can be commands which are particularly created for the present disclosure and the other part can be existing commands which are known to an ordinary person of a computer software field. The computer-readable medium can include a hardware device which is specially configured to store and execute the program commands. For example, the computer-readable record medium can become one of magnetic media including a hard disk, a floppy disk and a magnetic tape, an optical record media including CD-ROM and DVD, a magneto-optical record medium such as a floptical disk, and integrated circuit (IC) memories such as ROM, RAM and flash memory. As the program command, one of a machine language code made by a compiler and a high-level language code being executed by a computer using an interpreter can be employed. The hardware device can be replaced with at least one software module for performing operation procedures in accordance with the present disclosure and vice versa.

Specified execution procedures described in the present disclosure are introduced only as examples but can be implemented in any different manner. As such, the scope of the present disclosure is not limited to the examples. For the conciseness of description, electronic components, control systems, software and different functional sides thereof in accordance with the prior art can be omitted from the description. Also, connection lines or connection members between components shown in the drawings are represented as examples of functional connections and/or physical or circuitry connections, but can be really replaced with a variety of additionally functional connections, physical connections or circuitry connections. Moreover, components not definitely specified by terms of 'necessary', 'important' and so on cannot become essential components surely necessary to implement the present disclosure.

Although the present disclosure has been limitedly explained regarding only the embodiments described above, it should be understood by the person of ordinary skill or knowledge in the art that the present disclosure is not limited to these embodiments, but rather that various changes or modifications thereof are possible without departing from the spirit of the present disclosure. Accordingly, the technical scope of the present disclosure shall be determined only by the appended claims without being limited to the detailed description of the present disclosure.

### [Industrial Applicability]

The medical consultation support method and terminal in accordance with embodiment of the present disclosure can promote satisfaction at the medical consultation between the consultant and the consulter. As such, the medical consultation support method and terminal can be used in an industrial field.

## Claims

1. A method of supporting a medical consultation, the method comprising:
enabling a consultant to gain access to a medical consultation support program;
searching and selecting an image necessary to draft medical consultation content;
displaying a drafting page of the medical consultation content loaded with the selected image;
receiving input data of the consultant to be inserted in the drafting page of the medical consultation content;
generating the medical consultation content through a combination of the received input data and the drafting page of the medical consultation content; and
providing a consulter with the generated medical consultation content.

2. The method claimed as claim 1 further comprises displaying a list of the consulters, wherein the list of the consulters displays not only a part of the consulter being within a hospital in an activated state but also the other part of the consulter unreached the hospital in an inactivated state, on the basis of position information received from the consulters.

3. The method claimed as claim 1, wherein the input data of the consultant includes at least one of written data of the consultant input through a touch sensor and a consultation voice record recorded through a microphone.

4. The method claimed as claim 3, wherein the generation of the medical consultation content includes:
storing the written data of the consultant in an animation shape with the lapse of time;
recording the consultation voices with the lapse of time; and
combining the written data animation and the consultation voice record with the drafting page of the medical consultation content, and
provides a live clinic function.

5. The method claimed as claim 4, wherein the input data of the consultant further includes images obtained by photographing a consultation scene at a separate position and the medical consultation content further includes the photographed images.

6. The method claimed as claim 1, wherein
the input data of the consultant includes written data of the consultant input through a touch sensor, and
the generation of the medical consultation content includes turning the written data of the consultant at a previously set angle and combining the turned written data with the drafting page of the medical consultation content and provides a viewing direction change function.

7. The method claimed as claim 1, wherein the receipt of the input data of the consultant performs one of ignoring the input data received from the consultant and excluding the received input data of the consultant from the drafting page of the medical consultation content, at an execution of a privacy protection function.

8. The method claimed as claim 1, wherein the medical consultation content includes at least one of disease diagnosis content for explaining a disease of the consulter, medical treatment method content for explaining a medical treatment method for the consulter, medicinal prescription content for explaining medicines prescribed to the consulter, cost description content for explaining medical treatment costs of the consulter, insurance information content including insurance-related information, signature content, and survey content for a post marketing surveillance.

9. The method claimed as claim 8 further comprises generating the medical consultation content by unifying a plurality of consultation content in a generation sequence of the plurality of consultation content when the plurality of consultation content are generated.

10. The method claimed as claim 8, wherein
the medicinal consultation content include a drafting page of the medicinal consultation content with blanks which will be filled with medicine prescription numerals, and
the method further comprises automatically calculating medicine prescription numerals on the basis of information about the consulter and disease diagnosis information and inserting the calculated medicine prescription numerals in the blanks of the drafting page of the medicinal consultation content.

11. The method claimed as claim 8 further comprises transforming the medical consultation content into printable medical consultation content,
wherein the transformation of the medical consultation content includes at least one of setting sequentially input written data into completely input written data, converting the recorded consultation voices into texts, and capturing important image from one of an animation and a motion image included in the medical consultation content to display the captured images as successive process images.

12. The method claimed as claim 11 further comprises printing out the transformed printable medical consultation content by transmitting the transformed printable medical consultation content to a printer.

13. The method claimed as claim 8 further comprises:
generating survey content, which includes a survey image for a post marketing surveillance and signature panels filled with signatures, for the post marketing surveillance through a edition of the medical consultation content; and
transmitting the generated survey content for the post marketing surveillance to a server of a pharmaceutical company.

14. The method claimed as claim 8 further comprises:
generating shareable medical consultation content through a deletion of privacy information from the medical consultation content; and
transmitting the generated shareable medical consultation content to an operating server and enabling the generated shareable medial consultation content to be shared with the other persons.

15. A medical consultation support terminal comprising:
a touch screen configured to display graphic images and receive input data;
a processor configured to execute a medical consultation support program; and
a memory configured to store commands which will be executed by the processor, wherein the processor controls
a consultant to gain access to the medical consultation support program,
a graphical user interface searching and selecting an image necessary for a draft of medical consultation content to be provided through the touch screen,
a drafting page of the medical consultation content with a selected image to be displayed on the touch screen,
the input data, which will be inserted in the drafting page of the medical consultation content, to be received from the consultant through the touch screen,
the medical consultation content to be generated by combining the input data of the consultant and the drafting page of the medical consultation content, and
the generated medical consultation content to be transferred to a consulter.
